(19) Europäisches Patentamt
European Patent Office
Office européen des brevets

(11) EP 2 440 935 B1

(12) **EUROPEAN PATENT SPECIFICATION**

(45) Date of publication and mention of the grant of the patent:
**09.08.2017 Bulletin 2017/32**

(21) Application number: **10726041.6**

(22) Date of filing: **07.06.2010**

(51) Int Cl.:
***G01N 33/68*** *(2006.01)*

(86) International application number:
**PCT/EP2010/057917**

(87) International publication number:
**WO 2010/142637 (16.12.2010 Gazette 2010/50)**

(54) **A METHOD OF DIAGNOSING RENAL DISORDERS**

VERFAHREN ZUR DIAGNOSE VON NIERENERKRANKUNGEN

PROCÉDÉ DE DIAGNOSTIC DE TROUBLES RÉNAUX

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **09.06.2009 EP 09162296**

(43) Date of publication of application:
**18.04.2012 Bulletin 2012/16**

(73) Proprietor: **Mayer, Gert**
**6020 Innsbruck (AT)**

(72) Inventors:
- **RUDNICKI, Michael**
  **6020 Innsbruck (AT)**
- **PERCO, Paul**
  **1080 Vienna (AT)**

(74) Representative: **Loidl, Manuela Bettina et al**
**REDL Life Science Patent Attorneys**
**Donau-City-Straße 11**
**1220 Wien (AT)**

(56) References cited:
**WO-A-91/08230** **WO-A-2009/061368**
**WO-A1-2009/127644**

- **STOKES M B ET AL: "Up-regulation of extracellular matrix proteoglycans and collagen type I in human crescentic glomerulonephritis." KIDNEY INTERNATIONAL FEB 2001, vol. 59, no. 2, February 2001 (2001-02), pages 532-542, XP002544320 ISSN: 0085-2538**
- **DOURS-ZIMMERMANN M T ET AL: "A novel glycosaminoglycan attachment domain identified in two alternative splice variants of human versican." THE JOURNAL OF BIOLOGICAL CHEMISTRY 30 DEC 1994, vol. 269, no. 52, 30 December 1994 (1994-12-30), pages 32992-32998, XP002544321 ISSN: 0021-9258**
- **CATTARUZZA S ET AL: "Distribution of PG-M/versican variants in human tissues and de novo expression of isoform V3 upon endothelial cell activation, migration, and neoangiogenesis in vitro" JOURNAL OF BIOLOGICAL CHEMISTRY, AMERICAN SOCIETY OF BIOLOCHEMICAL BIOLOGISTS, BIRMINGHAM, US, vol. 277, no. 49, 6 December 2002 (2002-12-06), pages 47626-47635, XP003021624 ISSN: 0021-9258**
- **ARSLAN F ET AL: "The role of versican isoforms V0/V1 in glioma migration mediated by transforming growth factor-beta2." BRITISH JOURNAL OF CANCER 21 MAY 2007, vol. 96, no. 10, 21 May 2007 (2007-05-21), pages 1560-1568, XP002544322 ISSN: 0007-0920**
- **SASAMURA H ET AL: "Effects of AT1 receptor antagonist on proteoglycan gene expression in hypertensive rats" HYPERTENSION RESEARCH. CLINICAL AND EXPERIMENTAL, OSAKA, JP, vol. 24, no. 2, 1 January 2001 (2001-01-01), pages 165-172, XP003015666 ISSN: 0916-9636**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Mayer, Gert

<120> A method of diagnosing renal disorders

<130> NB4/P

<160> 3

<170> PatentIn version 3.3

<210> 1
<211> 3396
<212> PRT
<213> Homo sapiens

<400> 1

```
Met Phe Ile Asn Ile Lys Ser Ile Leu Trp Met Cys Ser Thr Leu Ile
1               5                   10                  15

Val Thr His Ala Leu His Lys Val Lys Val Gly Lys Ser Pro Pro Val
            20                  25                  30

Arg Gly Ser Leu Ser Gly Lys Val Ser Leu Pro Cys His Phe Ser Thr
        35                  40                  45

Met Pro Thr Leu Pro Pro Ser Tyr Asn Thr Ser Glu Phe Leu Arg Ile
    50                  55                  60

Lys Trp Ser Lys Ile Glu Val Asp Lys Asn Gly Lys Asp Leu Lys Glu
65                  70                  75                  80

Thr Thr Val Leu Val Ala Gln Asn Gly Asn Ile Lys Ile Gly Gln Asp
                85                  90                  95

Tyr Lys Gly Arg Val Ser Val Pro Thr His Pro Glu Ala Val Gly Asp
            100                 105                 110

Ala Ser Leu Thr Val Val Lys Leu Leu Ala Ser Asp Ala Gly Leu Tyr
        115                 120                 125

Arg Cys Asp Val Met Tyr Gly Ile Glu Asp Thr Gln Asp Thr Val Ser
    130                 135                 140

Leu Thr Val Asp Gly Val Val Phe His Tyr Arg Ala Ala Thr Ser Arg
145                 150                 155                 160

Tyr Thr Leu Asn Phe Glu Ala Ala Gln Lys Ala Cys Leu Asp Val Gly
                165                 170                 175
```

```
Ala Val Ile Ala Thr Pro Glu Gln Leu Phe Ala Ala Tyr Glu Asp Gly
            180                 185                 190

Phe Glu Gln Cys Asp Ala Gly Trp Leu Ala Asp Gln Thr Val Arg Tyr
        195                 200                 205

Pro Ile Arg Ala Pro Arg Val Gly Cys Tyr Gly Asp Lys Met Gly Lys
    210                 215                 220

Ala Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Pro Lys Glu Ala Thr
            340                 345                 350

Thr Ile Asp Leu Ser Ile Leu Ala Glu Thr Ala Ser Pro Ser Leu Ser
        355                 360                 365

Lys Glu Pro Gln Met Val Ser Asp Arg Thr Thr Pro Ile Ile Pro Leu
    370                 375                 380

Val Asp Glu Leu Pro Val Ile Pro Thr Glu Phe Pro Pro Val Gly Asn
385                 390                 395                 400

Ile Val Ser Phe Glu Gln Lys Ala Thr Val Gln Pro Gln Ala Ile Thr
                405                 410                 415

Asp Ser Leu Ala Thr Lys Leu Pro Thr Pro Thr Gly Ser Thr Lys Lys
            420                 425                 430
```

Pro Trp Asp Met Asp Asp Tyr Ser Pro Ser Ala Ser Gly Pro Leu Gly
435 440 445

Lys Leu Asp Ile Ser Glu Ile Lys Glu Glu Val Leu Gln Ser Thr Thr
450 455 460

Gly Val Ser His Tyr Ala Thr Asp Ser Trp Asp Gly Val Val Glu Asp
465 470 475 480

Lys Gln Thr Gln Glu Ser Val Thr Gln Ile Glu Gln Ile Glu Val Gly
485 490 495

Pro Leu Val Thr Ser Met Glu Ile Leu Lys His Ile Pro Ser Lys Glu
500 505 510

Phe Pro Val Thr Glu Thr Pro Leu Val Thr Ala Arg Met Ile Leu Glu
515 520 525

Ser Lys Thr Glu Lys Lys Met Val Ser Thr Val Ser Glu Leu Val Thr
530 535 540

Thr Gly His Tyr Gly Phe Thr Leu Gly Glu Glu Asp Asp Glu Asp Arg
545 550 555 560

Thr Leu Thr Val Gly Ser Asp Glu Ser Thr Leu Ile Phe Asp Gln Ile
565 570 575

Pro Glu Val Ile Thr Val Ser Lys Thr Ser Glu Asp Thr Ile His Thr
580 585 590

His Leu Glu Asp Leu Glu Ser Val Ser Ala Ser Thr Thr Val Ser Pro
595 600 605

Leu Ile Met Pro Asp Asn Asn Gly Ser Ser Met Asp Asp Trp Glu Glu
610 615 620

Arg Gln Thr Ser Gly Arg Ile Thr Glu Glu Phe Leu Gly Lys Tyr Leu
625 630 635 640

Ser Thr Thr Pro Phe Pro Ser Gln His Arg Thr Glu Ile Glu Leu Phe
645 650 655

Pro Tyr Ser Gly Asp Lys Ile Leu Val Glu Gly Ile Ser Thr Val Ile
660 665 670

Tyr Pro Ser Leu Gln Thr Glu Met Thr His Arg Arg Glu Arg Thr Glu
675 680 685

Thr Leu Ile Pro Glu Met Arg Thr Asp Thr Tyr Thr Asp Glu Ile Gln
690 695 700

Glu Glu Ile Thr Lys Ser Pro Phe Met Gly Lys Thr Glu Glu Glu Val
705 710 715 720

Phe Ser Gly Met Lys Leu Ser Thr Ser Leu Ser Glu Pro Ile His Val
725 730 735

Thr Glu Ser Ser Val Glu Met Thr Lys Ser Phe Asp Phe Pro Thr Leu
740 745 750

Ile Thr Lys Leu Ser Ala Glu Pro Thr Glu Val Arg Asp Met Glu Glu
755 760 765

Asp Phe Thr Ala Thr Pro Gly Thr Thr Lys Tyr Asp Glu Asn Ile Thr
770 775 780

Thr Val Leu Leu Ala His Gly Thr Leu Ser Val Glu Ala Ala Thr Val
785 790 795 800

Ser Lys Trp Ser Trp Asp Glu Asp Asn Thr Thr Ser Lys Pro Leu Glu
805 810 815

Ser Thr Glu Pro Ser Ala Ser Ser Lys Leu Pro Pro Ala Leu Leu Thr
820 825 830

Thr Val Gly Met Asn Gly Lys Asp Lys Asp Ile Pro Ser Phe Thr Glu
835 840 845

Asp Gly Ala Asp Glu Phe Thr Leu Ile Pro Asp Ser Thr Gln Lys Gln
850 855 860

Leu Glu Glu Val Thr Asp Glu Asp Ile Ala Ala His Gly Lys Phe Thr
865 870 875 880

Ile Arg Phe Gln Pro Thr Thr Ser Thr Gly Ile Ala Glu Lys Ser Thr
885 890 895

Leu Arg Asp Ser Thr Thr Glu Glu Lys Val Pro Pro Ile Thr Ser Thr
900 905 910

Glu Gly Gln Val Tyr Ala Thr Met Glu Gly Ser Ala Leu Gly Glu Val
915 920 925

Glu Asp Val Asp Leu Ser Lys Pro Val Ser Thr Val Pro Gln Phe Ala

930 935 940

His Thr Ser Glu Val Glu Gly Leu Ala Phe Val Ser Tyr Ser Ser Thr
945 950 955 960

Gln Glu Pro Thr Thr Tyr Val Asp Ser Ser His Thr Ile Pro Leu Ser
965 970 975

Val Ile Pro Lys Thr Asp Trp Gly Val Leu Val Pro Ser Val Pro Ser
980 985 990

Glu Asp Glu Val Leu Gly Glu Pro Ser Gln Asp Ile Leu Val Ile Asp
995 1000 1005

Gln Thr Arg Leu Glu Ala Thr Ile Ser Pro Glu Thr Met Arg Thr
1010 1015 1020

Thr Lys Ile Thr Glu Gly Thr Thr Gln Glu Glu Phe Pro Trp Lys
1025 1030 1035

Glu Gln Thr Ala Glu Lys Pro Val Pro Ala Leu Ser Ser Thr Ala
1040 1045 1050

Trp Thr Pro Lys Glu Ala Val Thr Pro Leu Asp Glu Gln Glu Gly
1055 1060 1065

Asp Gly Ser Ala Tyr Thr Val Ser Glu Asp Glu Leu Leu Thr Gly
1070 1075 1080

Ser Glu Arg Val Pro Val Leu Glu Thr Thr Pro Val Gly Lys Ile
1085 1090 1095

Asp His Ser Val Ser Tyr Pro Pro Gly Ala Val Thr Glu His Lys
1100 1105 1110

Val Lys Thr Asp Glu Val Val Thr Leu Thr Pro Arg Ile Gly Pro
1115 1120 1125

Lys Val Ser Leu Ser Pro Gly Pro Glu Gln Lys Tyr Glu Thr Glu
1130 1135 1140

Gly Ser Ser Thr Thr Gly Phe Thr Ser Ser Leu Ser Pro Phe Ser
1145 1150 1155

Thr His Ile Thr Gln Leu Met Glu Glu Thr Thr Thr Glu Lys Thr
1160 1165 1170

Ser Leu Glu Asp Ile Asp Leu Gly Ser Gly Leu Phe Glu Lys Pro
1175 1180 1185

Lys Ala Thr Glu Leu Ile Glu Phe Ser Thr Ile Lys Val Thr Val
1190 1195 1200

Pro Ser Asp Ile Thr Thr Ala Phe Ser Ser Val Asp Arg Leu His
1205 1210 1215

Thr Thr Ser Ala Phe Lys Pro Ser Ser Ala Ile Thr Lys Lys Pro
1220 1225 1230

Pro Leu Ile Asp Arg Glu Pro Gly Glu Glu Thr Thr Ser Asp Met
1235 1240 1245

Val Ile Ile Gly Glu Ser Thr Ser His Val Pro Pro Thr Thr Leu
1250 1255 1260

Glu Asp Ile Val Ala Lys Glu Thr Glu Thr Asp Ile Asp Arg Glu
1265 1270 1275

Tyr Phe Thr Thr Ser Ser Pro Pro Ala Thr Gln Pro Thr Arg Pro
1280 1285 1290

Pro Thr Val Glu Asp Lys Glu Ala Phe Gly Pro Gln Ala Leu Ser
1295 1300 1305

Thr Pro Gln Pro Pro Ala Ser Thr Lys Phe His Pro Asp Ile Asn
1310 1315 1320

Val Tyr Ile Ile Glu Val Arg Glu Asn Lys Thr Gly Arg Met Ser
1325 1330 1335

Asp Leu Ser Val Ile Gly His Pro Ile Asp Ser Glu Ser Lys Glu
1340 1345 1350

Asp Glu Pro Cys Ser Glu Glu Thr Asp Pro Val His Asp Leu Met
1355 1360 1365

Ala Glu Ile Leu Pro Glu Phe Pro Asp Ile Ile Glu Ile Asp Leu
1370 1375 1380

Tyr His Ser Glu Glu Asn Glu Glu Glu Glu Glu Glu Cys Ala Asn
1385 1390 1395

Ala Thr Asp Val Thr Thr Thr Pro Ser Val Gln Tyr Ile Asn Gly
1400 1405 1410

```
Lys His Leu Val Thr Thr Val Pro Lys Asp Pro Glu Ala Ala Glu
    1415                1420                1425

Ala Arg Arg Gly Gln Phe Glu Ser Val Ala Pro Ser Gln Asn Phe
    1430                1435                1440

Ser Asp Ser Ser Glu Ser Asp Thr His Pro Phe Val Ile Ala Lys
    1445                1450                1455

Thr Glu Leu Ser Thr Ala Val Gln Pro Asn Glu Ser Thr Glu Thr
    1460                1465                1470

Thr Glu Ser Leu Glu Val Thr Trp Lys Pro Glu Thr Tyr Pro Glu
    1475                1480                1485

Thr Ser Glu His Phe Ser Gly Gly Glu Pro Asp Val Phe Pro Thr
    1490                1495                1500

Val Pro Phe His Glu Glu Phe Glu Ser Gly Thr Ala Lys Lys Gly
    1505                1510                1515

Ala Glu Ser Val Thr Glu Arg Asp Thr Glu Val Gly His Gln Ala
    1520                1525                1530

His Glu His Thr Glu Pro Val Ser Leu Phe Pro Glu Glu Ser Ser
    1535                1540                1545

Gly Glu Ile Ala Ile Asp Gln Glu Ser Gln Lys Ile Ala Phe Ala
    1550                1555                1560

Arg Ala Thr Glu Val Thr Phe Gly Glu Glu Val Glu Lys Ser Thr
    1565                1570                1575

Ser Val Thr Tyr Thr Pro Thr Ile Val Pro Ser Ser Ala Ser Ala
    1580                1585                1590

Tyr Val Ser Glu Glu Glu Ala Val Thr Leu Ile Gly Asn Pro Trp
    1595                1600                1605

Pro Asp Asp Leu Leu Ser Thr Lys Glu Ser Trp Val Glu Ala Thr
    1610                1615                1620

Pro Arg Gln Val Val Glu Leu Ser Gly Ser Ser Ser Ile Pro Ile
    1625                1630                1635

Thr Glu Gly Ser Gly Glu Ala Glu Glu Asp Glu Asp Thr Met Phe
    1640                1645                1650
```

```
Thr Met  Val Thr Asp Leu Ser  Gln Arg Asn Thr Thr  Asp Thr Leu
    1655                 1660                 1665

Ile Thr  Leu Asp Thr Ser Arg  Ile Ile Thr Glu Ser  Phe Phe Glu
    1670                 1675                 1680

Val Pro  Ala Thr Thr Ile Tyr  Pro Val Ser Glu Gln  Pro Ser Ala
    1685                 1690                 1695

Lys Val  Val Pro Thr Lys Phe  Val Ser Glu Thr Asp  Thr Ser Glu
    1700                 1705                 1710

Trp Ile  Ser Ser Thr Thr Val  Glu Glu Lys Lys Arg  Lys Glu Glu
    1715                 1720                 1725

Glu Gly  Thr Thr Gly Thr Ala  Ser Thr Phe Glu Val  Tyr Ser Ser
    1730                 1735                 1740

Thr Gln  Arg Ser Asp Gln Leu  Ile Leu Pro Phe Glu  Leu Glu Ser
    1745                 1750                 1755

Pro Asn  Val Ala Thr Ser Ser  Asp Ser Gly Thr Arg  Lys Ser Phe
    1760                 1765                 1770

Met Ser  Leu Thr Thr Pro Thr  Gln Ser Glu Arg Glu  Met Thr Asp
    1775                 1780                 1785

Ser Thr  Pro Val Phe Thr Glu  Thr Asn Thr Leu Glu  Asn Leu Gly
    1790                 1795                 1800

Ala Gln  Thr Thr Glu His Ser  Ser Ile His Gln Pro  Gly Val Gln
    1805                 1810                 1815

Glu Gly  Leu Thr Thr Leu Pro  Arg Ser Pro Ala Ser  Val Phe Met
    1820                 1825                 1830

Glu Gln  Gly Ser Gly Glu Ala  Ala Ala Asp Pro Glu  Thr Thr Thr
    1835                 1840                 1845

Val Ser  Ser Phe Ser Leu Asn  Val Glu Tyr Ala Ile  Gln Ala Glu
    1850                 1855                 1860

Lys Glu  Val Ala Gly Thr Leu  Ser Pro His Val Glu  Thr Thr Phe
    1865                 1870                 1875

Ser Thr  Glu Pro Thr Gly Leu  Val Leu Ser Thr Val  Met Asp Arg
```

1880 1885 1890

Val Val Ala Glu Asn Ile Thr Gln Thr Ser Arg Glu Ile Val Ile
1895 1900 1905

Ser Glu Arg Leu Gly Glu Pro Asn Tyr Gly Ala Glu Ile Arg Gly
1910 1915 1920

Phe Ser Thr Gly Phe Pro Leu Glu Glu Asp Phe Ser Gly Asp Phe
1925 1930 1935

Arg Glu Tyr Ser Thr Val Ser His Pro Ile Ala Lys Glu Glu Thr
1940 1945 1950

Val Met Met Glu Gly Ser Gly Asp Ala Ala Phe Arg Asp Thr Gln
1955 1960 1965

Thr Ser Pro Ser Thr Val Pro Thr Ser Val His Ile Ser His Ile
1970 1975 1980

Ser Asp Ser Glu Gly Pro Ser Ser Thr Met Val Ser Thr Ser Ala
1985 1990 1995

Phe Pro Trp Glu Glu Phe Thr Ser Ser Ala Glu Gly Ser Gly Glu
2000 2005 2010

Gln Leu Val Thr Val Ser Ser Ser Val Val Pro Val Leu Pro Ser
2015 2020 2025

Ala Val Gln Lys Phe Ser Gly Thr Ala Ser Ser Ile Ile Asp Glu
2030 2035 2040

Gly Leu Gly Glu Val Gly Thr Val Asn Glu Ile Asp Arg Arg Ser
2045 2050 2055

Thr Ile Leu Pro Thr Ala Glu Val Glu Gly Thr Lys Ala Pro Val
2060 2065 2070

Glu Lys Glu Glu Val Lys Val Ser Gly Thr Val Ser Thr Asn Phe
2075 2080 2085

Pro Gln Thr Ile Glu Pro Ala Lys Leu Trp Ser Arg Gln Glu Val
2090 2095 2100

Asn Pro Val Arg Gln Glu Ile Glu Ser Glu Thr Thr Ser Glu Glu
2105 2110 2115

Gln Ile Gln Glu Glu Lys Ser Phe Glu Ser Pro Gln Asn Ser Pro
2120 2125 2130

Ala Thr Glu Gln Thr Ile Phe Asp Ser Gln Thr Phe Thr Glu Thr
2135 2140 2145

Glu Leu Lys Thr Thr Asp Tyr Ser Val Leu Thr Thr Lys Lys Thr
2150 2155 2160

Tyr Ser Asp Asp Lys Glu Met Lys Glu Glu Asp Thr Ser Leu Val
2165 2170 2175

Asn Met Ser Thr Pro Asp Pro Asp Ala Asn Gly Leu Glu Ser Tyr
2180 2185 2190

Thr Thr Leu Pro Glu Ala Thr Glu Lys Ser His Phe Phe Leu Ala
2195 2200 2205

Thr Ala Leu Val Thr Glu Ser Ile Pro Ala Glu His Val Val Thr
2210 2215 2220

Asp Ser Pro Ile Lys Lys Glu Glu Ser Thr Lys His Phe Pro Lys
2225 2230 2235

Gly Met Arg Pro Thr Ile Gln Glu Ser Asp Thr Glu Leu Leu Phe
2240 2245 2250

Ser Gly Leu Gly Ser Gly Glu Glu Val Leu Pro Thr Leu Pro Thr
2255 2260 2265

Glu Ser Val Asn Phe Thr Glu Val Glu Gln Ile Asn Asn Thr Leu
2270 2275 2280

Tyr Pro His Thr Ser Gln Val Glu Ser Thr Ser Ser Asp Lys Ile
2285 2290 2295

Glu Asp Phe Asn Arg Met Glu Asn Val Ala Lys Glu Val Gly Pro
2300 2305 2310

Leu Val Ser Gln Thr Asp Ile Phe Glu Gly Ser Gly Ser Val Thr
2315 2320 2325

Ser Thr Thr Leu Ile Glu Ile Leu Ser Asp Thr Gly Ala Glu Gly
2330 2335 2340

Pro Thr Val Ala Pro Leu Pro Phe Ser Thr Asp Ile Gly His Pro
2345 2350 2355

```
Gln Asn Gln Thr Val Arg Trp Ala Glu Glu Ile Gln Thr Ser Arg
    2360                2365                2370

Pro Gln Thr Ile Thr Glu Gln Asp Ser Asn Lys Asn Ser Ser Thr
    2375                2380                2385

Ala Glu Ile Asn Glu Thr Thr Thr Ser Ser Thr Asp Phe Leu Ala
    2390                2395                2400

Arg Ala Tyr Gly Phe Glu Met Ala Lys Glu Phe Val Thr Ser Ala
    2405                2410                2415

Pro Lys Pro Ser Asp Leu Tyr Tyr Glu Pro Ser Gly Glu Gly Ser
    2420                2425                2430

Gly Glu Val Asp Ile Val Asp Ser Phe His Thr Ser Ala Thr Thr
    2435                2440                2445

Gln Ala Thr Arg Gln Glu Ser Ser Thr Thr Phe Val Ser Asp Gly
    2450                2455                2460

Ser Leu Glu Lys His Pro Glu Val Pro Ser Ala Lys Ala Val Thr
    2465                2470                2475

Ala Asp Gly Phe Pro Thr Val Ser Val Met Leu Pro Leu His Ser
    2480                2485                2490

Glu Gln Asn Lys Ser Ser Pro Asp Pro Thr Ser Thr Leu Ser Asn
    2495                2500                2505

Thr Val Ser Tyr Glu Arg Ser Thr Asp Gly Ser Phe Gln Asp Arg
    2510                2515                2520

Phe Arg Glu Phe Glu Asp Ser Thr Leu Lys Pro Asn Arg Lys Lys
    2525                2530                2535

Pro Thr Glu Asn Ile Ile Ile Asp Leu Asp Lys Glu Asp Lys Asp
    2540                2545                2550

Leu Ile Leu Thr Ile Thr Glu Ser Thr Ile Leu Glu Ile Leu Pro
    2555                2560                2565

Glu Leu Thr Ser Asp Lys Asn Thr Ile Ile Asp Ile Asp His Thr
    2570                2575                2580

Lys Pro Val Tyr Glu Asp Ile Leu Gly Met Gln Thr Asp Ile Asp
    2585                2590                2595
```

Thr Glu Val Pro Ser Glu Pro His Asp Ser Asn Asp Glu Ser Asn
2600 2605 2610

Asp Asp Ser Thr Gln Val Gln Glu Ile Tyr Glu Ala Ala Val Asn
2615 2620 2625

Leu Ser Leu Thr Glu Glu Thr Phe Glu Gly Ser Ala Asp Val Leu
2630 2635 2640

Ala Ser Tyr Thr Gln Ala Thr His Asp Glu Ser Met Thr Tyr Glu
2645 2650 2655

Asp Arg Ser Gln Leu Asp His Met Gly Phe His Phe Thr Thr Gly
2660 2665 2670

Ile Pro Ala Pro Ser Thr Glu Thr Glu Leu Asp Val Leu Leu Pro
2675 2680 2685

Thr Ala Thr Ser Leu Pro Ile Pro Arg Lys Ser Ala Thr Val Ile
2690 2695 2700

Pro Glu Ile Glu Gly Ile Lys Ala Glu Ala Lys Ala Leu Asp Asp
2705 2710 2715

Met Phe Glu Ser Ser Thr Leu Ser Asp Gly Gln Ala Ile Ala Asp
2720 2725 2730

Gln Ser Glu Ile Ile Pro Thr Leu Gly Gln Phe Glu Arg Thr Gln
2735 2740 2745

Glu Glu Tyr Glu Asp Lys Lys His Ala Gly Pro Ser Phe Gln Pro
2750 2755 2760

Glu Phe Ser Ser Gly Ala Glu Glu Ala Leu Val Asp His Thr Pro
2765 2770 2775

Tyr Leu Ser Ile Ala Thr Thr His Leu Met Asp Gln Ser Val Thr
2780 2785 2790

Glu Val Pro Asp Val Met Glu Gly Ser Asn Pro Pro Tyr Tyr Thr
2795 2800 2805

Asp Thr Thr Leu Ala Val Ser Thr Phe Ala Lys Leu Ser Ser Gln
2810 2815 2820

Thr Pro Ser Ser Pro Leu Thr Ile Tyr Ser Gly Ser Glu Ala Ser

```
    2825                2830                2835

Gly His  Thr Glu Ile Pro Gln  Pro Ser Ala Leu Pro  Gly Ile Asp
    2840                2845                2850

Val Gly  Ser Ser Val Met Ser  Pro Gln Asp Ser Phe  Lys Glu Ile
    2855                2860                2865

His Val  Asn Ile Glu Ala Thr  Phe Lys Pro Ser Ser  Glu Glu Tyr
    2870                2875                2880

Leu His  Ile Thr Glu Pro Pro  Ser Leu Ser Pro Asp  Thr Lys Leu
    2885                2890                2895

Glu Pro  Ser Glu Asp Asp Gly  Lys Pro Glu Leu Leu  Glu Glu Met
    2900                2905                2910

Glu Ala  Ser Pro Thr Glu Leu  Ile Ala Val Glu Gly  Thr Glu Ile
    2915                2920                2925

Leu Gln  Asp Phe Gln Asn Lys  Thr Asp Gly Gln Val  Ser Gly Glu
    2930                2935                2940

Ala Ile  Lys Met Phe Pro Thr  Ile Lys Thr Pro Glu  Ala Gly Thr
    2945                2950                2955

Val Ile  Thr Thr Ala Asp Glu  Ile Glu Leu Glu Gly  Ala Thr Gln
    2960                2965                2970

Trp Pro  His Ser Thr Ser Ala  Ser Ala Thr Tyr Gly  Val Glu Ala
    2975                2980                2985

Gly Val  Val Pro Trp Leu Ser  Pro Gln Thr Ser Glu  Arg Pro Thr
    2990                2995                3000

Leu Ser  Ser Ser Pro Glu Ile  Asn Pro Glu Thr Gln  Ala Ala Leu
    3005                3010                3015

Ile Arg  Gly Gln Asp Ser Thr  Ile Ala Ala Ser Glu  Gln Gln Val
    3020                3025                3030

Ala Ala  Arg Ile Leu Asp Ser  Asn Asp Gln Ala Thr  Val Asn Pro
    3035                3040                3045

Val Glu  Phe Asn Thr Glu Val  Ala Thr Pro Pro Phe  Ser Leu Leu
    3050                3055                3060
```

Glu Thr Ser Asn Glu Thr Asp Phe Leu Ile Gly Ile Asn Glu Glu
3065 3070 3075

Ser Val Glu Gly Thr Ala Ile Tyr Leu Pro Gly Pro Asp Arg Cys
3080 3085 3090

Lys Met Asn Pro Cys Leu Asn Gly Gly Thr Cys Tyr Pro Thr Glu
3095 3100 3105

Thr Ser Tyr Val Cys Thr Cys Val Pro Gly Tyr Ser Gly Asp Gln
3110 3115 3120

Cys Glu Leu Asp Phe Asp Glu Cys His Ser Asn Pro Cys Arg Asn
3125 3130 3135

Gly Ala Thr Cys Val Asp Gly Phe Asn Thr Phe Arg Cys Leu Cys
3140 3145 3150

Leu Pro Ser Tyr Val Gly Ala Leu Cys Glu Gln Asp Thr Glu Thr
3155 3160 3165

Cys Asp Tyr Gly Trp His Lys Phe Gln Gly Gln Cys Tyr Lys Tyr
3170 3175 3180

Phe Ala His Arg Arg Thr Trp Asp Ala Ala Glu Arg Glu Cys Arg
3185 3190 3195

Leu Gln Gly Ala His Leu Thr Ser Ile Leu Ser His Glu Glu Gln
3200 3205 3210

Met Phe Val Asn Arg Val Gly His Asp Tyr Gln Trp Ile Gly Leu
3215 3220 3225

Asn Asp Lys Met Phe Glu His Asp Phe Arg Trp Thr Asp Gly Ser
3230 3235 3240

Thr Leu Gln Tyr Glu Asn Trp Arg Pro Asn Gln Pro Asp Ser Phe
3245 3250 3255

Phe Ser Ala Gly Glu Asp Cys Val Val Ile Ile Trp His Glu Asn
3260 3265 3270

Gly Gln Trp Asn Asp Val Pro Cys Asn Tyr His Leu Thr Tyr Thr
3275 3280 3285

Cys Lys Lys Gly Thr Val Ala Cys Gly Gln Pro Pro Val Val Glu
3290 3295 3300

```
Asn Ala  Lys Thr Phe Gly Lys  Met Lys Pro Arg Tyr  Glu Ile Asn
    3305                 3310                 3315

Ser Leu  Ile Arg Tyr His Cys  Lys Asp Gly Phe Ile  Gln Arg His
    3320                 3325                 3330

Leu Pro  Thr Ile Arg Cys Leu  Gly Asn Gly Arg Trp  Ala Ile Pro
    3335                 3340                 3345

Lys Ile  Thr Cys Met Asn Pro  Ser Ala Tyr Gln Arg  Thr Tyr Ser
    3350                 3355                 3360

Met Lys  Tyr Phe Lys Asn Ser  Ser Ser Ala Lys Asp  Asn Ser Ile
    3365                 3370                 3375

Asn Thr  Ser Lys His Asp His  Arg Trp Ser Arg Arg  Trp Gln Glu
    3380                 3385                 3390

Ser Arg  Arg
    3395
```

<210> 2
<211> 12057
<212> DNA
<213> Homo sapiens

<400> 2

```
acagtgatat aatgatgatg ggtgtcacaa cccgcatttg aacttgcagg cgagctgccc      60
cgagcctttc tggggaagaa ctccaggcgt gcggacgcaa cagccgagaa cattaggtgt     120
tgtggacagg agctgggacc aagatcttcg gccagccccg catcctcccg catcttccag     180
caccgtcccg caccctccgc atccttcccc gggccaccac gcttcctatg tgacccgcct     240
gggcaacgcc gaacccagtc gcgcagcgct gcagtgaatt ttccccccaa actgcaataa     300
gccgccttcc aaggccaaga tgttcataaa tataaagagc atcttatgga tgtgttcaac     360
cttaatagta acccatgcgc tacataaagt caaagtggga aaaagcccac cggtgagggg     420
ctccctctct ggaaaagtca gcctaccttg tcatttttca acgatgccta ctttgccacc     480
cagttacaac accagtgaat ttctccgcat caaatggtct aagattgaag tggacaaaaa     540
tggaaaagat ttgaaagaga ctactgtcct tgtggcccaa aatggaaata tcaagattgg     600
tcaggactac aaagggagag tgtctgtgcc cacacatccc gaggctgtgg gcgatgcctc     660
cctcactgtg gtcaagctgc tggcaagtga tgcgggtctt taccgctgtg acgtcatgta     720
cgggattgaa gacacacaag acacggtgtc actgactgtg gatggggttg tgtttcacta     780
cagggcggca accagcaggt acacactgaa ttttgaggct gctcagaagg cttgtttgga     840
```

cgttggggca gtcatagcaa ctccagagca gctctttgct gcctatgaag atggatttga 900

gcagtgtgac gcaggctggc tggctgatca gactgtcaga tatcccatcc gggctcccag 960

agtaggctgt tatggagata agatgggaaa ggcaggagtc aggacttatg gattccgttc 1020

tccccaggaa acttacgatg tgtattgtta tgtggatcat ctggatggtg atgtgttcca 1080

cctcactgtc cccagtaaat tcaccttcga ggaggctgca aaagagtgtg aaaaccagga 1140

tgccaggctg gcaacagtgg gggaactcca ggcggcatgg aggaacggct ttgaccagtg 1200

cgattacggg tggctgtcgg atgccagcgt gcgccaccct gtgactgtgg ccagggccca 1260

gtgtggaggt ggtctacttg ggtgagaac cctgtatcgt tttgagaacc agacaggctt 1320

ccctccccct gatagcagat ttgatgccta ctgctttaaa cctaaagagg ctacaaccat 1380

cgatttgagt atcctcgcag aaactgcatc acccagttta tccaaagaac cacaaatggt 1440

ttctgataga actacaccaa tcatcccttt agttgatgaa ttacctgtca ttccaacaga 1500

gttccctccc gtgggaaata ttgtcagttt tgaacagaaa gccacagtcc aacctcaggc 1560

tatcacagat agtttagcca ccaaattacc cacacctact ggcagtacca agaagccctg 1620

ggatatggat gactactcac cttctgcttc aggacctctt ggaaagctag acatatcaga 1680

aattaaggaa gaagtgctcc agagtacaac tggcgtctct cattatgcta cggattcatg 1740

ggatggtgtc gtggaagata aacaaacaca agaatcggtt acacagattg aacaaataga 1800

agtgggtcct ttggtaacat ctatggaaat cttaaagcac attccttcca aggaattccc 1860

tgtaactgaa acaccattgg taactgcaag aatgatcctg gaatccaaaa ctgaaaagaa 1920

aatggtaagc actgtttctg aattggtaac cacaggtcac tatggattca ccttgggaga 1980

agaggatgat gaagacagaa cacttacagt tggatctgat gagagcacct tgatctttga 2040

ccaaattcct gaagtcatta cggtgtcaaa gacttcagaa gacaccatcc acactcattt 2100

agaagacttg gagtcagtct cagcatccac aactgtttcc cctttaatta tgcctgataa 2160

taatggatca tccatggatg actgggaaga gagacaaact agtggtagga taacggaaga 2220

gtttcttggc aaatatctgt ctactacacc ttttccatca cagcatcgta cagaaataga 2280

attgtttcct tattctggtg ataaaatatt agtagaggga atttccacag ttatttatcc 2340

ttctctacaa acagaaatga cacatagaag agaaagaaca gaaacactaa taccagagat 2400

gagaacagat acttatacag atgaaataca agaagagatc actaaaagtc catttatggg 2460

aaaaacagaa gaagaagtct tctctgggat gaaactctct acatctctct cagagccaat 2520

tcatgttaca gagtcttctg tggaaatgac caagtctttt gatttcccaa cattgataac 2580

aaagttaagt gcagagccaa cagaagtaag agatatggag gaagacttta cagcaactcc 2640

aggtactaca aaatatgatg aaaatattac aacagtgctt ttggcccatg gtactttaag 2700

tgttgaagca gccactgtat caaaatggtc atgggatgaa gataatacaa catccaagcc 2760

```
tttagagtct acagaacctt cagcctcttc aaaattgccc cctgccttac tcacaactgt   2820
ggggatgaat ggaaaggata aagacatccc aagtttcact gaagatggag cagatgaatt   2880
tactcttatt ccagatagta ctcaaaagca gttagaggag gttactgatg aagacatagc   2940
agcccatgga aaattcacaa ttagatttca gccaactaca tcaactggta ttgcagaaaa   3000
gtcaactttg agagattcta caactgaaga aaaagttcca cctatcacaa gcactgaagg   3060
ccaagtttat gcaaccatgg aaggaagtgc tttgggtgaa gtagaagatg tggacctctc   3120
taagccagta tctactgttc cccaatttgc acacacttca gaggtggaag gattagcatt   3180
tgttagttat agtagcaccc aagagcctac tacttatgta gactcttccc ataccattcc   3240
tctttctgta attcccaaga cagactgggg agtgttagta ccttctgttc catcagaaga   3300
tgaagttcta ggtgaaccct ctcaagacat acttgtcatt gatcagactc gccttgaagc   3360
gactatttct ccagaaacta tgagaacaac aaaaatcaca gagggaacaa ctcaggaaga   3420
attcccttgg aaagaacaga ctgcagagaa accagttcct gctctcagtt ctacagcttg   3480
gactcccaag gaggcagtaa caccactgga tgaacaagag ggcgatggat cagcatatac   3540
agtctctgaa gatgaattgt tgacaggttc tgagagggtc ccagttttag aaacaactcc   3600
agttggaaaa attgatcaca gtgtgtctta tccaccaggt gctgtaactg agcacaaagt   3660
gaaaacagat gaagtggtaa cactaacacc acgcattggg ccaaaagtat ctttaagtcc   3720
agggcctgaa caaaaatatg aaacagaagg tagtagtaca acaggattta catcatcttt   3780
gagtcctttt agtacccaca ttacccagct tatggaagaa accactactg agaaaacatc   3840
cctagaggat attgatttag gctcaggatt atttgaaaag cccaaagcca cagaactcat   3900
agaattttca acaatcaaag tcacagttcc aagtgatatt accactgcct tcagttcagt   3960
agacagactt cacacaactt cagcattcaa gccatcttcc gcgatcacta agaaaccacc   4020
tctcatcgac agggaacctg gtgaagaaac aaccagtgac atggtaatca ttggagaatc   4080
aacatctcat gttcctccca ctacccttga agatattgta gccaaggaaa cagaaaccga   4140
tattgataga gagtatttca cgacttcaag tcctcctgct acacagccaa caagaccacc   4200
cactgtggaa gacaaagagg cctttggacc tcaggcgctt tctacgccac agcccccagc   4260
aagcacaaaa tttcaccctg acattaatgt ttatattatt gaggtcagag aaaataagac   4320
aggtcgaatg agtgatttga gtgtaattgg tcatccaata gattcagaat ctaaagaaga   4380
tgaaccttgt agtgaagaaa cagatccagt gcatgatcta atggctgaaa ttttacctga   4440
attccctgac ataattgaaa tagacctata ccacagtgaa gaaaatgaag aagaagaaga   4500
agagtgtgca aatgctactg atgtgacaac caccccatct gtgcagtaca taaatgggaa   4560
gcatctcgtt accactgtgc ccaaggaccc agaagctgca gaagctaggc gtggccagtt   4620
```

tgaaagtgtt gcaccttctc agaatttctc ggacagctct gaaagtgata ctcatccatt 4680
tgtaatagcc aaaacggaat tgtctactgc tgtgcaacct aatgaatcta cagaaacaac 4740
tgagtctctt gaagttacat ggaagcctga gacttaccct gaaacatcag aacatttttc 4800
aggtggtgag cctgatgttt tccccacagt cccattccat gaggaatttg aaagtggaac 4860
agccaaaaaa ggggcagaat cagtcacaga gagagatact gaagttggtc atcaggcaca 4920
tgaacatact gaacctgtat ctctgtttcc tgaagagtct tcaggagaga ttgccattga 4980
ccaagaatct cagaaaatag cctttgcaag ggctacagaa gtaacatttg gtgaagaggt 5040
agaaaaaagt acttctgtca catacactcc cactatagtt ccaagttctg catcagcata 5100
tgtttcagag gaagaagcag ttaccctaat aggaaatcct tggccagatg acctgttgtc 5160
taccaaagaa agctgggtag aagcaactcc tagacaagtt gtagagctct cagggagttc 5220
ttcgattcca attacagaag gctctggaga agcagaagaa gatgaagata caatgttcac 5280
catggtaact gatttatcac agagaaatac tactgataca ctcattactt tagacactag 5340
caggataatc acagaaagct tttttgaggt tcctgcaacc accatttatc cagtttctga 5400
acaaccttct gcaaaagtgg tgcctaccaa gtttgtaagt gaaacagaca cttctgagtg 5460
gatttccagt accactgttg aggaaaagaa aaggaaggag gaggagggaa ctacaggtac 5520
ggcttctaca tttgaggtat attcatctac acagagatcg gatcaattaa ttttaccctt 5580
tgaattagaa agtccaaatg tagctacatc tagtgattca ggtaccagga aaagttttat 5640
gtccttgaca acaccaacac agtctgaaag ggaaatgaca gattctactc ctgtctttac 5700
agaaacaaat acattagaaa atttgggggc acagaccact gagcacagca gtatccatca 5760
acctggggtt caggaagggc tgaccactct cccacgtagt cctgcctctg tctttatgga 5820
gcagggctct ggagaagctg ctgccgaccc agaaaccacc actgtttctt cattttcatt 5880
aaacgtagag tatgcaattc aagccgaaaa ggaagtagct ggcactttgt ctccgcatgt 5940
ggaaactaca ttctccactg agccaacagg actggttttg agtacagtaa tggacagagt 6000
agttgctgaa aatataaccc aaacatccag ggaaatagtg atttcagagc gattaggaga 6060
accaaattat ggggcagaaa taagggggctt ttccacaggt tttcctttgg aggaagattt 6120
cagtggtgac tttagagaat actcaacagt gtctcatccc atagcaaaag aagaaacggt 6180
aatgatggaa ggctctggag atgcagcatt tagggacacc cagacttcac catctacagt 6240
acctacttca gttcacatca gtcacatatc tgactcagaa ggacccagta gcaccatggt 6300
cagcacttca gccttcccct gggaagagtt tacatcctca gctgagggct caggtgagca 6360
actggtcaca gtcagcagct ctgttgttcc agtgcttccc agtgctgtgc aaaagttttc 6420
tggtacagct tcctccatta tcgacgaagg attgggagaa gtgggtactg tcaatgaaat 6480
tgatagaaga tccaccattt taccaacagc agaagtggaa ggtacgaaag ctccagtaga 6540

gaaggaggaa gtaaaggtca gtggcacagt ttcaacaaac tttccccaaa ctatagagcc 6600
agccaaatta tggtctaggc aagaagtcaa ccctgtaaga caagaaattg aaagtgaaac 6660
aacatcagag gaacaaattc aagaagaaaa gtcatttgaa tcccctcaaa actctcctgc 6720
aacagaacaa acaatctttg attcacagac atttactgaa actgaactca aaaccacaga 6780
ttattctgta ctaacaacaa agaaaactta cagtgatgat aaagaaatga aggaggaaga 6840
cacttcttta gttaacatgt ctactccaga tccagatgca aatggcttgg aatcttacac 6900
aactctccct gaagctactg aaaagtcaca ttttttctta gctactgcat tagtaactga 6960
atctatacca gctgaacatg tagtcacaga ttcaccaatc aaaaaggaag aaagtacaaa 7020
acattttccg aaaggcatga gaccaacaat tcaagagtca gatactgagc tcttattctc 7080
tggactggga tcaggagaag aagttttacc tactctacca acagagtcag tgaattttac 7140
tgaagtggaa caaatcaata acacattata tccccacact tctcaagtgg aaagtacctc 7200
aagtgacaaa attgaagact ttaacagaat ggaaaatgtg gcaaaagaag ttggaccact 7260
cgtatctcaa acagacatct ttgaaggtag tgggtcagta accagcacaa cattaataga 7320
aattttaagt gacactggag cagaaggacc cacggtggca cctctccctt tctccacgga 7380
catcggacat cctcaaaatc agactgtcag gtgggcagaa gaaatccaga ctagtagacc 7440
acaaaccata actgaacaag actctaacaa gaattcttca acagcagaaa ttaacgaaac 7500
aacaacctca tctactgatt ttctggctag agcttatggt tttgaaatgg ccaaagaatt 7560
tgttacatca gcaccaaaac catctgactt gtattatgaa ccttctggag aaggatctgg 7620
agaagtggat attgttgatt catttcacac ttctgcaact actcaggcaa ccagacaaga 7680
aagcagcacc acatttgttt ctgatgggtc cctggaaaaa catcctgagg tgccaagcgc 7740
taaagctgtt actgctgatg gattcccaac agtttcagtg atgctgcctc ttcattcaga 7800
gcagaacaaa agctcccctg atccaactag cacactgtca aatacagtgt catatgagag 7860
gtccacagac ggtagtttcc aagaccgttt cagggaattc gaggattcca ccttaaaacc 7920
taacagaaaa aaacccactg aaaatattat catagacctg gacaaagagg acaaggattt 7980
aatattgaca attacagaga gtaccatcct tgaaattcta cctgagctga catcggataa 8040
aaatactatc atagatattg atcatactaa acctgtgtat gaagacattc ttggaatgca 8100
aacagatata gatacagagg taccatcaga accacatgac agtaatgatg aaagtaatga 8160
tgacagcact caagttcaag agatctatga ggcagctgtc aacctttctt taactgagga 8220
aacatttgag ggctctgctg atgttctggc tagctacact caggcaacac atgatgaatc 8280
aatgacttat gaagatagaa gccaactaga tcacatgggc tttcacttca caactgggat 8340
ccctgctcct agcacagaaa cagaattaga cgttttactt cccacggcaa catccctgcc 8400

aattcctcgt aagtctgcca cagttattcc agagattgaa ggaataaaag ctgaagcaaa 8460
agccctggat gacatgtttg aatcaagcac tttgtctgat ggtcaagcta ttgcagacca 8520
aagtgaaata ataccaacat tgggccaatt tgaaaggact caggaggagt atgaagacaa 8580
aaaacatgct ggtccttctt ttcagccaga attctcttca ggagctgagg aggcattagt 8640
agaccatact ccctatctaa gtattgctac tacccacctt atggatcaga gtgtaacaga 8700
ggtgcctgat gtgatggaag gatccaatcc cccatattac actgatacaa cattagcagt 8760
ttcaacattt gcgaagttgt cttctcagac accatcatct cccctcacta tctactcagg 8820
cagtgaagcc tctggacaca cagagatccc ccagcccagt gctctgccag gaatagacgt 8880
cggctcatct gtaatgtccc cacaggattc ttttaaggaa attcatgtaa atattgaagc 8940
gactttcaaa ccatcaagtg aggaatacct tcacataact gagcctccct ctttatctcc 9000
tgacacaaaa ttagaacctt cagaagatga tggtaaacct gagttattag aagaaatgga 9060
agcttctccc acagaactta ttgctgtgga aggaactgag attctccaag atttccaaaa 9120
caaaaccgat ggtcaagttt ctggagaagc aatcaagatg tttcccacca ttaaaacacc 9180
tgaggctgga actgttatta caactgccga tgaaattgaa ttagaaggtg ctacacagtg 9240
gccacactct acttctgctt ctgccaccta tggggtcgag gcaggtgtgg tgccttggct 9300
aagtccacag acttctgaga ggcccacgct ttcttcttct ccagaaataa accctgaaac 9360
tcaagcagct ttaatcagag ggcaggattc cacgatagca gcatcagaac agcaagtggc 9420
agcgagaatt cttgattcca atgatcaggc aacagtaaac cctgtggaat ttaatactga 9480
ggttgcaaca ccaccatttt cccttctgga gacttctaat gaaacagatt tcctgattgg 9540
cattaatgaa gagtcagtgg aaggcacggc aatctattta ccaggacctg atcgctgcaa 9600
aatgaacccg tgccttaacg gaggcacctg ttatcctact gaaacttcct acgtatgcac 9660
ctgtgtgcca ggatacagcg gagaccagtg tgaacttgat tttgatgaat gtcactctaa 9720
tccctgtcgt aatggagcca cttgtgttga tggttttaac acattcaggt gcctctgcct 9780
tccaagttat gttggtgcac tttgtgagca agataccgag acatgtgact atggctggca 9840
caaattccaa gggcagtgct acaaatactt tgcccatcga cgcacatggg atgcagctga 9900
acgggaatgc cgtctgcagg gtgcccatct cacaagcatc ctgtctcacg aagaacaaat 9960
gtttgttaat cgtgtgggcc atgattatca gtggataggc ctcaatgaca agatgtttga 10020
gcatgacttc cgttggactg atggcagcac actgcaatac gagaattgga gacccaacca 10080
gccagacagc ttcttttctg ctggagaaga ctgtgttgta atcatttggc atgagaatgg 10140
ccagtggaat gatgttccct gcaattacca tctcacctat acgtgcaaga aaggaacagt 10200
cgcttgcggc cagccccctg ttgtagaaaa tgccaagacc tttggaaaga tgaaacctcg 10260
ttatgaaatc aactccctga ttagatacca ctgcaaagat ggtttcattc aacgtcacct 10320

tccaactatc cggtgcttag gaaatggaag atgggctata cctaaaatta cctgcatgaa 10380
cccatctgca taccaaagga cttattctat gaaatacttt aaaaattcct catcagcaaa 10440
ggacaattca ataaatacat ccaaacatga tcatcgttgg agccggaggt ggcaggagtc 10500
gaggcgctga tccctaaaat ggcgaacatg tgttttcatc atttcagcca aagtcctaac 10560
ttcctgtgcc tttcctatca cctcgagaag taattatcag ttggtttgga tttttggacc 10620
accgttcagt cattttgggt tgccgtgctc ccaaaacatt ttaaatgaaa gtattggcat 10680
tcaaaaagac agcagacaaa atgaaagaaa atgagagcag aaagtaagca tttccagcct 10740
atctaatttc tttagttttc tatttgcctc cagtgcagtc catttcctaa tgtataccag 10800
cctactgtac tatttaaaat gctcaatttc agcaccgatg gccatgtaaa taagatgatt 10860
taatgttgat tttaatcctg tatataaaat aaaaagtcac aatgagtttg ggcatattta 10920
atgatgatta tggagcctta gaggtcttta atcattggtt cggctgcttt tatgtagttt 10980
aggctggaaa tggtttcact tgctctttga ctgtcagcaa gactgaagat ggcttttcct 11040
ggacagctag aaaacacaaa atcttgtagg tcattgcacc tatctcagcc ataggtgcag 11100
tttgcttcta catgatgcta aaggctgcga atgggatcct gatggaacta aggactccaa 11160
tgtcgaactc ttctttgctg cattcctttt tcttcactta caagaaaggc ctgaatggag 11220
gacttttctg taaccaggaa cattttttag gggtcaaagt gctaataatt aactcaacca 11280
ggtctacttt ttaatggctt tcataacact aactcataag gttaccgatc aatgcatttc 11340
atacggatat agacctaggg ctctggaggg tgggggattg ttaaaacaca tgcaaaaaaa 11400
aaaaaaaaaa aaaaaaaaga aattttgtat atataaccat tttaatcttt tataaagttt 11460
tgaatgttca tgtatgaatg ctgcagctgt gaagcataca taaataaatg aagtaagcca 11520
tactgattta atttattgga tgttattttc cctaagacct gaaaatgaac atagtatgct 11580
agttattttt cagtgttagc cttttacttt cctcacacaa tttggaatca tataatatag 11640
gtactttgtc cctgattaaa taatgtgacg gatagaatgc atcaagtgtt tattatgaaa 11700
agagtggaaa agtatatagc ttttagcaaa aggtgtttgc ccattctaag aaatgagcga 11760
atatatagaa atagtgtggg catttcttcc tgttaggtgg agtgtatgtg ttgacatttc 11820
tccccatctc ttcccactct gttttctccc cattatttga ataaagtgac tgctgaagat 11880
gactttgaat ccttatccac ttaatttaat gtttaaagaa aaacctgtaa tggaaagtaa 11940
gactccttcc ctaatttcag tttagagcaa cttgaagaag agtagacaaa aaataaaatg 12000
cacatagaaa aagagaaaaa gggcacaaag ggattggccc aatattgatt ctttttt 12057

<210> 3
<211> 2409
<212> PRT

<213> Homo sapiens

<400> 3

Met Phe Ile Asn Ile Lys Ser Ile Leu Trp Met Cys Ser Thr Leu Ile
1 5 10 15

Val Thr His Ala Leu His Lys Val Lys Val Gly Lys Ser Pro Pro Val
20 25 30

Arg Gly Ser Leu Ser Gly Lys Val Ser Leu Pro Cys His Phe Ser Thr
35 40 45

Met Pro Thr Leu Pro Pro Ser Tyr Asn Thr Ser Glu Phe Leu Arg Ile
50 55 60

Lys Trp Ser Lys Ile Glu Val Asp Lys Asn Gly Lys Asp Leu Lys Glu
65 70 75 80

Thr Thr Val Leu Val Ala Gln Asn Gly Asn Ile Lys Ile Gly Gln Asp
85 90 95

Tyr Lys Gly Arg Val Ser Val Pro Thr His Pro Glu Ala Val Gly Asp
100 105 110

Ala Ser Leu Thr Val Val Lys Leu Leu Ala Ser Asp Ala Gly Leu Tyr
115 120 125

Arg Cys Asp Val Met Tyr Gly Ile Glu Asp Thr Gln Asp Thr Val Ser
130 135 140

Leu Thr Val Asp Gly Val Val Phe His Tyr Arg Ala Ala Thr Ser Arg
145 150 155 160

Tyr Thr Leu Asn Phe Glu Ala Ala Gln Lys Ala Cys Leu Asp Val Gly
165 170 175

Ala Val Ile Ala Thr Pro Glu Gln Leu Phe Ala Ala Tyr Glu Asp Gly
180 185 190

Phe Glu Gln Cys Asp Ala Gly Trp Leu Ala Asp Gln Thr Val Arg Tyr
195 200 205

Pro Ile Arg Ala Pro Arg Val Gly Cys Tyr Gly Asp Lys Met Gly Lys
210 215 220

Ala Gly Val Arg Thr Tyr Gly Phe Arg Ser Pro Gln Glu Thr Tyr Asp
225 230 235 240

```
Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Arg Arg Met Ser Asp
            340                 345                 350

Leu Ser Val Ile Gly His Pro Ile Asp Ser Glu Ser Lys Glu Asp Glu
        355                 360                 365

Pro Cys Ser Glu Glu Thr Asp Pro Val His Asp Leu Met Ala Glu Ile
    370                 375                 380

Leu Pro Glu Phe Pro Asp Ile Ile Glu Ile Asp Leu Tyr His Ser Glu
385                 390                 395                 400

Glu Asn Glu Glu Glu Glu Glu Glu Cys Ala Asn Ala Thr Asp Val Thr
                405                 410                 415

Thr Thr Pro Ser Val Gln Tyr Ile Asn Gly Lys His Leu Val Thr Thr
            420                 425                 430

Val Pro Lys Asp Pro Glu Ala Ala Glu Ala Arg Arg Gly Gln Phe Glu
        435                 440                 445

Ser Val Ala Pro Ser Gln Asn Phe Ser Asp Ser Ser Glu Ser Asp Thr
    450                 455                 460

His Pro Phe Val Ile Ala Lys Thr Glu Leu Ser Thr Ala Val Gln Pro
465                 470                 475                 480

Asn Glu Ser Thr Glu Thr Thr Glu Ser Leu Glu Val Thr Trp Lys Pro
                485                 490                 495
```

```
Glu Thr Tyr Pro Glu Thr Ser Glu His Phe Ser Gly Gly Glu Pro Asp
            500                 505                 510

Val Phe Pro Thr Val Pro Phe His Glu Glu Phe Glu Ser Gly Thr Ala
        515                 520                 525

Lys Lys Gly Ala Glu Ser Val Thr Glu Arg Asp Thr Glu Val Gly His
    530                 535                 540

Gln Ala His Glu His Thr Glu Pro Val Ser Leu Phe Pro Glu Glu Ser
545                 550                 555                 560

Ser Gly Glu Ile Ala Ile Asp Gln Glu Ser Gln Lys Ile Ala Phe Ala
                565                 570                 575

Arg Ala Thr Glu Val Thr Phe Gly Glu Glu Val Glu Lys Ser Thr Ser
            580                 585                 590

Val Thr Tyr Thr Pro Thr Ile Val Pro Ser Ser Ala Ser Ala Tyr Val
        595                 600                 605

Ser Glu Glu Glu Ala Val Thr Leu Ile Gly Asn Pro Trp Pro Asp Asp
    610                 615                 620

Leu Leu Ser Thr Lys Glu Ser Trp Val Glu Ala Thr Pro Arg Gln Val
625                 630                 635                 640

Val Glu Leu Ser Gly Ser Ser Ser Ile Pro Ile Thr Glu Gly Ser Gly
                645                 650                 655

Glu Ala Glu Glu Asp Glu Asp Thr Met Phe Thr Met Val Thr Asp Leu
            660                 665                 670

Ser Gln Arg Asn Thr Thr Asp Thr Leu Ile Thr Leu Asp Thr Ser Arg
        675                 680                 685

Ile Ile Thr Glu Ser Phe Phe Glu Val Pro Ala Thr Thr Ile Tyr Pro
    690                 695                 700

Val Ser Glu Gln Pro Ser Ala Lys Val Val Pro Thr Lys Phe Val Ser
705                 710                 715                 720

Glu Thr Asp Thr Ser Glu Trp Ile Ser Ser Thr Thr Val Glu Glu Lys
                725                 730                 735

Lys Arg Lys Glu Glu Glu Gly Thr Thr Gly Thr Ala Ser Thr Phe Glu
```

740 745 750

Val Tyr Ser Ser Thr Gln Arg Ser Asp Gln Leu Ile Leu Pro Phe Glu
755 760 765

Leu Glu Ser Pro Asn Val Ala Thr Ser Ser Asp Ser Gly Thr Arg Lys
770 775 780

Ser Phe Met Ser Leu Thr Thr Pro Thr Gln Ser Glu Arg Glu Met Thr
785 790 795 800

Asp Ser Thr Pro Val Phe Thr Glu Thr Asn Thr Leu Glu Asn Leu Gly
805 810 815

Ala Gln Thr Thr Glu His Ser Ser Ile His Gln Pro Gly Val Gln Glu
820 825 830

Gly Leu Thr Thr Leu Pro Arg Ser Pro Ala Ser Val Phe Met Glu Gln
835 840 845

Gly Ser Gly Glu Ala Ala Ala Asp Pro Glu Thr Thr Thr Val Ser Ser
850 855 860

Phe Ser Leu Asn Val Glu Tyr Ala Ile Gln Ala Glu Lys Glu Val Ala
865 870 875 880

Gly Thr Leu Ser Pro His Val Glu Thr Thr Phe Ser Thr Glu Pro Thr
885 890 895

Gly Leu Val Leu Ser Thr Val Met Asp Arg Val Val Ala Glu Asn Ile
900 905 910

Thr Gln Thr Ser Arg Glu Ile Val Ile Ser Glu Arg Leu Gly Glu Pro
915 920 925

Asn Tyr Gly Ala Glu Ile Arg Gly Phe Ser Thr Gly Phe Pro Leu Glu
930 935 940

Glu Asp Phe Ser Gly Asp Phe Arg Glu Tyr Ser Thr Val Ser His Pro
945 950 955 960

Ile Ala Lys Glu Glu Thr Val Met Met Glu Gly Ser Gly Asp Ala Ala
965 970 975

Phe Arg Asp Thr Gln Thr Ser Pro Ser Thr Val Pro Thr Ser Val His
980 985 990

```
Ile Ser His Ile Ser Asp Ser Glu  Gly Pro Ser Ser Thr  Met Val Ser
        995                 1000                1005

Thr Ser  Ala Phe Pro Trp Glu  Glu Phe Thr Ser Ser  Ala Glu Gly
    1010                 1015                 1020

Ser Gly  Glu Gln Leu Val Thr  Val Ser Ser Ser Val  Val Pro Val
    1025                 1030                 1035

Leu Pro  Ser Ala Val Gln Lys  Phe Ser Gly Thr Ala  Ser Ser Ile
    1040                 1045                 1050

Ile Asp  Glu Gly Leu Gly Glu  Val Gly Thr Val Asn  Glu Ile Asp
    1055                 1060                 1065

Arg Arg  Ser Thr Ile Leu Pro  Thr Ala Glu Val Glu  Gly Thr Lys
    1070                 1075                 1080

Ala Pro  Val Glu Lys Glu Glu  Val Lys Val Ser Gly  Thr Val Ser
    1085                 1090                 1095

Thr Asn  Phe Pro Gln Thr Ile  Glu Pro Ala Lys Leu  Trp Ser Arg
    1100                 1105                 1110

Gln Glu  Val Asn Pro Val Arg  Gln Glu Ile Glu Ser  Glu Thr Thr
    1115                 1120                 1125

Ser Glu  Glu Gln Ile Gln Glu  Glu Lys Ser Phe Glu  Ser Pro Gln
    1130                 1135                 1140

Asn Ser  Pro Ala Thr Glu Gln  Thr Ile Phe Asp Ser  Gln Thr Phe
    1145                 1150                 1155

Thr Glu  Thr Glu Leu Lys Thr  Thr Asp Tyr Ser Val  Leu Thr Thr
    1160                 1165                 1170

Lys Lys  Thr Tyr Ser Asp Asp  Lys Glu Met Lys Glu  Glu Asp Thr
    1175                 1180                 1185

Ser Leu  Val Asn Met Ser Thr  Pro Asp Pro Asp Ala  Asn Gly Leu
    1190                 1195                 1200

Glu Ser  Tyr Thr Thr Leu Pro  Glu Ala Thr Glu Lys  Ser His Phe
    1205                 1210                 1215

Phe Leu  Ala Thr Ala Leu Val  Thr Glu Ser Ile Pro  Ala Glu His
    1220                 1225                 1230
```

```
Val Val  Thr Asp Ser Pro Ile  Lys Lys Glu Glu Ser  Thr Lys His
    1235                 1240                 1245

Phe Pro  Lys Gly Met Arg Pro  Thr Ile Gln Glu Ser  Asp Thr Glu
    1250                 1255                 1260

Leu Leu  Phe Ser Gly Leu Gly  Ser Gly Glu Glu Val  Leu Pro Thr
    1265                 1270                 1275

Leu Pro  Thr Glu Ser Val Asn  Phe Thr Glu Val Glu  Gln Ile Asn
    1280                 1285                 1290

Asn Thr  Leu Tyr Pro His Thr  Ser Gln Val Glu Ser  Thr Ser Ser
    1295                 1300                 1305

Asp Lys  Ile Glu Asp Phe Asn  Arg Met Glu Asn Val  Ala Lys Glu
    1310                 1315                 1320

Val Gly  Pro Leu Val Ser Gln  Thr Asp Ile Phe Glu  Gly Ser Gly
    1325                 1330                 1335

Ser Val  Thr Ser Thr Thr Leu  Ile Glu Ile Leu Ser  Asp Thr Gly
    1340                 1345                 1350

Ala Glu  Gly Pro Thr Val Ala  Pro Leu Pro Phe Ser  Thr Asp Ile
    1355                 1360                 1365

Gly His  Pro Gln Asn Gln Thr  Val Arg Trp Ala Glu  Glu Ile Gln
    1370                 1375                 1380

Thr Ser  Arg Pro Gln Thr Ile  Thr Glu Gln Asp Ser  Asn Lys Asn
    1385                 1390                 1395

Ser Ser  Thr Ala Glu Ile Asn  Glu Thr Thr Thr Ser  Ser Thr Asp
    1400                 1405                 1410

Phe Leu  Ala Arg Ala Tyr Gly  Phe Glu Met Ala Lys  Glu Phe Val
    1415                 1420                 1425

Thr Ser  Ala Pro Lys Pro Ser  Asp Leu Tyr Tyr Glu  Pro Ser Gly
    1430                 1435                 1440

Glu Gly  Ser Gly Glu Val Asp  Ile Val Asp Ser Phe  His Thr Ser
    1445                 1450                 1455

Ala Thr  Thr Gln Ala Thr Arg  Gln Glu Ser Ser Thr  Thr Phe Val
    1460                 1465                 1470
```

Ser Asp Gly Ser Leu Glu Lys His Pro Glu Val Pro Ser Ala Lys
1475 1480 1485

Ala Val Thr Ala Asp Gly Phe Pro Thr Val Ser Val Met Leu Pro
1490 1495 1500

Leu His Ser Glu Gln Asn Lys Ser Ser Pro Asp Pro Thr Ser Thr
1505 1510 1515

Leu Ser Asn Thr Val Ser Tyr Glu Arg Ser Thr Asp Gly Ser Phe
1520 1525 1530

Gln Asp Arg Phe Arg Glu Phe Glu Asp Ser Thr Leu Lys Pro Asn
1535 1540 1545

Arg Lys Lys Pro Thr Glu Asn Ile Ile Ile Asp Leu Asp Lys Glu
1550 1555 1560

Asp Lys Asp Leu Ile Leu Thr Ile Thr Glu Ser Thr Ile Leu Glu
1565 1570 1575

Ile Leu Pro Glu Leu Thr Ser Asp Lys Asn Thr Ile Ile Asp Ile
1580 1585 1590

Asp His Thr Lys Pro Val Tyr Glu Asp Ile Leu Gly Met Gln Thr
1595 1600 1605

Asp Ile Asp Thr Glu Val Pro Ser Glu Pro His Asp Ser Asn Asp
1610 1615 1620

Glu Ser Asn Asp Asp Ser Thr Gln Val Gln Glu Ile Tyr Glu Ala
1625 1630 1635

Ala Val Asn Leu Ser Leu Thr Glu Glu Thr Phe Glu Gly Ser Ala
1640 1645 1650

Asp Val Leu Ala Ser Tyr Thr Gln Ala Thr His Asp Glu Ser Met
1655 1660 1665

Thr Tyr Glu Asp Arg Ser Gln Leu Asp His Met Gly Phe His Phe
1670 1675 1680

Thr Thr Gly Ile Pro Ala Pro Ser Thr Glu Thr Glu Leu Asp Val
1685 1690 1695

Leu Leu Pro Thr Ala Thr Ser Leu Pro Ile Pro Arg Lys Ser Ala

1700 1705 1710

Thr Val Ile Pro Glu Ile Glu Gly Ile Lys Ala Glu Ala Lys Ala
1715 1720 1725

Leu Asp Asp Met Phe Glu Ser Ser Thr Leu Ser Asp Gly Gln Ala
1730 1735 1740

Ile Ala Asp Gln Ser Glu Ile Ile Pro Thr Leu Gly Gln Phe Glu
1745 1750 1755

Arg Thr Gln Glu Glu Tyr Glu Asp Lys Lys His Ala Gly Pro Ser
1760 1765 1770

Phe Gln Pro Glu Phe Ser Ser Gly Ala Glu Glu Ala Leu Val Asp
1775 1780 1785

His Thr Pro Tyr Leu Ser Ile Ala Thr Thr His Leu Met Asp Gln
1790 1795 1800

Ser Val Thr Glu Val Pro Asp Val Met Glu Gly Ser Asn Pro Pro
1805 1810 1815

Tyr Tyr Thr Asp Thr Thr Leu Ala Val Ser Thr Phe Ala Lys Leu
1820 1825 1830

Ser Ser Gln Thr Pro Ser Ser Pro Leu Thr Ile Tyr Ser Gly Ser
1835 1840 1845

Glu Ala Ser Gly His Thr Glu Ile Pro Gln Pro Ser Ala Leu Pro
1850 1855 1860

Gly Ile Asp Val Gly Ser Ser Val Met Ser Pro Gln Asp Ser Phe
1865 1870 1875

Lys Glu Ile His Val Asn Ile Glu Ala Thr Phe Lys Pro Ser Ser
1880 1885 1890

Glu Glu Tyr Leu His Ile Thr Glu Pro Pro Ser Leu Ser Pro Asp
1895 1900 1905

Thr Lys Leu Glu Pro Ser Glu Asp Asp Gly Lys Pro Glu Leu Leu
1910 1915 1920

Glu Glu Met Glu Ala Ser Pro Thr Glu Leu Ile Ala Val Glu Gly
1925 1930 1935

Thr Glu Ile Leu Gln Asp Phe Gln Asn Lys Thr Asp Gly Gln Val
1940 1945 1950

Ser Gly Glu Ala Ile Lys Met Phe Pro Thr Ile Lys Thr Pro Glu
1955 1960 1965

Ala Gly Thr Val Ile Thr Thr Ala Asp Glu Ile Glu Leu Glu Gly
1970 1975 1980

Ala Thr Gln Trp Pro His Ser Thr Ser Ala Ser Ala Thr Tyr Gly
1985 1990 1995

Val Glu Ala Gly Val Val Pro Trp Leu Ser Pro Gln Thr Ser Glu
2000 2005 2010

Arg Pro Thr Leu Ser Ser Ser Pro Glu Ile Asn Pro Glu Thr Gln
2015 2020 2025

Ala Ala Leu Ile Arg Gly Gln Asp Ser Thr Ile Ala Ala Ser Glu
2030 2035 2040

Gln Gln Val Ala Ala Arg Ile Leu Asp Ser Asn Asp Gln Ala Thr
2045 2050 2055

Val Asn Pro Val Glu Phe Asn Thr Glu Val Ala Thr Pro Pro Phe
2060 2065 2070

Ser Leu Leu Glu Thr Ser Asn Glu Thr Asp Phe Leu Ile Gly Ile
2075 2080 2085

Asn Glu Glu Ser Val Glu Gly Thr Ala Ile Tyr Leu Pro Gly Pro
2090 2095 2100

Asp Arg Cys Lys Met Asn Pro Cys Leu Asn Gly Gly Thr Cys Tyr
2105 2110 2115

Pro Thr Glu Thr Ser Tyr Val Cys Thr Cys Val Pro Gly Tyr Ser
2120 2125 2130

Gly Asp Gln Cys Glu Leu Asp Phe Asp Glu Cys His Ser Asn Pro
2135 2140 2145

Cys Arg Asn Gly Ala Thr Cys Val Asp Gly Phe Asn Thr Phe Arg
2150 2155 2160

Cys Leu Cys Leu Pro Ser Tyr Val Gly Ala Leu Cys Glu Gln Asp
2165 2170 2175

```
Thr Glu  Thr Cys Asp Tyr Gly  Trp His Lys Phe Gln  Gly Gln Cys
    2180                 2185                 2190

Tyr Lys  Tyr Phe Ala His Arg  Arg Thr Trp Asp Ala  Ala Glu Arg
    2195                 2200                 2205

Glu Cys  Arg Leu Gln Gly Ala  His Leu Thr Ser Ile  Leu Ser His
    2210                 2215                 2220

Glu Glu  Gln Met Phe Val Asn  Arg Val Gly His Asp  Tyr Gln Trp
    2225                 2230                 2235

Ile Gly  Leu Asn Asp Lys Met  Phe Glu His Asp Phe  Arg Trp Thr
    2240                 2245                 2250

Asp Gly  Ser Thr Leu Gln Tyr  Glu Asn Trp Arg Pro  Asn Gln Pro
    2255                 2260                 2265

Asp Ser  Phe Phe Ser Ala Gly  Glu Asp Cys Val Val  Ile Ile Trp
    2270                 2275                 2280

His Glu  Asn Gly Gln Trp Asn  Asp Val Pro Cys Asn  Tyr His Leu
    2285                 2290                 2295

Thr Tyr  Thr Cys Lys Lys Gly  Thr Val Ala Cys Gly  Gln Pro Pro
    2300                 2305                 2310

Val Val  Glu Asn Ala Lys Thr  Phe Gly Lys Met Lys  Pro Arg Tyr
    2315                 2320                 2325

Glu Ile  Asn Ser Leu Ile Arg  Tyr His Cys Lys Asp  Gly Phe Ile
    2330                 2335                 2340

Gln Arg  His Leu Pro Thr Ile  Arg Cys Leu Gly Asn  Gly Arg Trp
    2345                 2350                 2355

Ala Ile  Pro Lys Ile Thr Cys  Met Asn Pro Ser Ala  Tyr Gln Arg
    2360                 2365                 2370

Thr Tyr  Ser Met Lys Tyr Phe  Lys Asn Ser Ser Ser  Ala Lys Asp
    2375                 2380                 2385

Asn Ser  Ile Asn Thr Ser Lys  His Asp His Arg Trp  Ser Arg Arg
    2390                 2395                 2400

Trp Gln  Glu Ser Arg Arg
    2405
```

## Description

The present invention relates to a method for determining given renal disorders or the risk of developing renal disorders in a patient by measuring a VCAN parameter.

Renal disorders, also called nephropathies, are diverse, but individuals with kidney disease frequently display characteristic clinical features including the nephritic and nephrotic syndromes, acute kidney failure, chronic kidney disease, urinary tract infection, nephrolithiasis, and urinary tract obstruction.

Acute kidney injury (AKI) is in the clinical setting described as acute renal failure (ARF) or acute tubular necrosis (ATN) and refers to the spontaneous and significant decrease in renal function. AKI therefore reflects the entire spectrum of ARF, recognizing that an acute decline in kidney function is often secondary to an injury that causes functional or structural changes in the kidneys. ARF is a frequent and serious problem with a variety of adverse short- and long-term clinical consequences. Loss of function of the kidney, a vital organ, in the form of acute renal failure represents a special hazard, in particular to older patients, despite modern therapies including the use of the various forms of artificial kidney. In diagnosis and prognosis care must be taken to differentiate between functional renal insufficiency and intrinsic injury with morphologic damage.

AKI in particular in the intensive care unit is often associated with multiple organ failure and sepsis. Furthermore, AKI is associated with high mortality and morbidity in humans. Patients, for instance, experience AKI in ischemic reperfusion injury, along with treatment with nephrotoxic compounds including but not limited to antibiotics or anticancer drugs, application of contrast media e.g. when performing angiography resulting in nephropathy or nephrotoxicity, or at the intensive care unit, e.g. in the context of sepsis. The annual number of patients receiving contrast media is more than 100 million in the developed countries, and the rate of acute kidney injury ranges in a percent range, if coupled to risk factors like hypotension or diabetes.

AKI is usually categorised according to pre-renal, intrinsic and post-renal causes.

Pre-renal (causes in the blood supply):

- hypovolemia (decreased blood volume), usually from shock or dehydration and fluid loss or excessive diuretics use.
- hepatorenal syndrome, in which renal perfusion is compromised in liver failure
- vascular problems, such as atheroembolic disease and renal vein thrombosis (which can occur as a complication of the nephrotic syndrome)
- infection usually sepsis, systemic inflammation due to infection
- severe burns
- sequestration due to pericarditis and pancreatitis
- hypotension due to antihypertensives and vasodilators Intrinsic (damage to the kidney itself):
- toxins or medication (e.g. some NSAIDs, aminoglycoside antibiotics, iodinated contrast, lithium, phosphate nephropathy due to bowel preparation for colonoscopy with sodium phosphates)
- rhabdomyolysis (breakdown of muscle tissue) - the resultant release of myoglobin in the blood affects the kidney; it can be caused by injury (especially crush injury and extensive blunt trauma), statins, stimulants and some other drugs
- hemolysis (breakdown of red blood cells) - the hemoglobin damages the tubules; it may be caused by various conditions such as sickle-cell disease, and lupus erythematosus
- multiple myeloma, either due to hypercalcemia or "cast nephropathy" (multiple myeloma can also cause chronic renal failure by a different mechanism)
- acute glomerulonephritis which may be due to a variety of causes, such as anti glomerular basement membrane disease/Goodpasture's syndrome, Wegener's granulomatosis or acute lupus nephritis with systemic lupus erythematosus

Post-renal (obstructive causes in the urinary tract) due to:

- medication interfering with normal bladder emptying (e.g. anticholinergics).
- benign prostatic hypertrophy or prostate cancer.
- kidney stones.
- due to abdominal malignancy (e.g. ovarian cancer, colorectal cancer).
- obstructed urinary catheter.
- drugs that can cause crystalluria and drugs that can lead to myoglobinuria and cystitis

According to the state of the art, renal failure is diagnosed when either creatinine or blood urea nitrogen tests are markedly elevated in an ill patient, especially when oliguria is present. Previous measurements of renal function may offer comparison, which is especially important if a patient is known to have chronic renal failure as well. If the cause is not apparent, a large amount of blood tests and examination of a urine specimen is typically performed to elucidate the cause of acute renal failure, medical ultrasonography of the renal tract is essential to rule out obstruction of the urinary tract.

An exemplary consensus criterium for the diagnosis of AKI is at least one of the following:

- Risk: serum creatinine increased 1.5 times or urine production of less than 0.5 ml/kg body weight for 6 hours
- Injury: creatinine 2.0 times OR urine production less than 0.5 ml/kg for 12 h
- Failure: creatinine 3.0 times OR creatinine more than 355 μmol/l (with a rise of more than 44) or urine output below 0.3 ml/kg for 24 h
- Loss: persistent AKI or complete loss of kidney function for more than four weeks
- End-stage Renal Disease: complete loss of kidney function for more than three months.

A rapid increase in serum creatinine may also be an indicator for a high AKI risk following medical treatment, e.g. impairment in renal function is indicated by an increase in serum creatinine by more than 0.5 mg/dl or more than 25% within 3 days after medication.

Kidney biopsy may be performed in the setting of acute renal failure, to provide a definitive diagnosis and sometimes an idea of the prognosis, unless the cause is clear and appropriate screening investigations are reassuringly negative.

To diagnose AKI, usually urine and blood tests are done and the volume of urine produced is monitored.

The gold standard for diagnosing AKI is the measurement of serum creatinine. Unfortunately, creatinine as marker has several limitations. On the one hand, levels of serum creatinine widely vary among individuals depending on age, sex, muscle mass or medication status. On the other hand, serum creatinine does not accurately depict kidney function during acute changes in glomerular filtration as it is a marker, which can only be interpreted in steady state. Furthermore creatinine levels do not rise until damage is severe and kidney function already declines. Other biomarkers such as lipocalin 2 (LCN2), also known as NGAL (neutrophil gelatinase-associated lipocalin), kidney injury molecule 1 (KIM1), cysteine-rich angiogenic inducer 61 (CYR61), or interleukin 18 (IL18) have recently been proposed as alternative parameters for the detection of acute kidney injury.

Patients with normal kidney function are currently not tested for any renal disease biomarkers. In the absence of any functional kidney disorder, such as urine volume reduction or creatinine level, it is assumed that there is no risk for developing AKI. However, there are patients, who have the potential to develop AKI upon certain medical treatment, which could be damaging to the kidney function, such as simple radiography using a contrast medium or chemotherapy. Several risk factors for acute renal failure have been identified so far.

High-risk patients are considered those with chronic diseases that can affect the kidneys like diabetes, hypertension and heart disease. Pregnant patients who suffer from eclampsia, a hypertensive condition, also have a high risk for kidney damage.

Some drugs are nephrotoxic, i.e. poisonous to the kidney, and therefore damaging to the kidneys. This includes certain antibiotics like aminoglycosides, antiinflammatory drugs and the contrast media used in specific X-ray tests of the urinary tract. A need therefore exists for a marker which can be used to specifically and reproducibly detect the presence of, or predisposition to acquiring AKI clinically leading to ARF.

Chronic kidney disease (CKD) affects up to 13 % of the general population and its prevalence is steadily rising. Progressive loss of kidney function is accompanied by increased morbidity and mortality from cardiovascular disease and bone metabolism disorders, and the treatment of end-stage renal disease is a major healthcare challenge. Since the natural history of CKD shows a high intraindividual variation reliable histological and serological markers capable to differentiate between stable and progressive disease are heavily needed. Published data on biomarkers predicting progression are scarce, and their significance is often limited.

The increasing prevalence of patients on renal replacement therapy has become a major challenge for healthcare systems. Frequently, end stage renal disease (ESRD) is the terminal phase of a chronic process. A better understanding of the pathophysiology of progressive kidney disease could lead to the development of new treatment options which might be able to stabilize renal function and reduce the incidence of ESRD. In order to use new but also the already available drugs even more efficiently, it is also highly desirable to identify patients with an adverse renal prognosis in the early phases of the disease as not all subjects show a relentlessly progressive decline in renal function. In this context the magnitude of proteinuria has been suggested to be a useful risk marker, even though, on an individual basis, the discriminatory power is questionable. Other biomarkers such as apolipoprotein A-IV (APOA4), adiponectin (ADIPOQ), or fibroblast growth factor 23 (FGF23) have recently been proposed as alternative parameters to predict the course of disease.

Cardiovascular disease (CVD) is a major cause of morbidity and mortality in patients suffering from chronic kidney disease. Around 50% of deaths of patients with end-stage renal disease are caused by cardiovascular complications. At the same time almost all patients with ESRD show signs of renal osteodystrophy, a heterogeneous pattern of bone metabolism disorders caused by chronic renal insufficiency and concomitant diseases.

The elevated risk of CVD in chronic kidney patients is partly based on traditional risk factors such as hypertension or diabetes mellitus. Next to these traditional risk factors a number of biomarker candidates are discussed in the scientific literature to be predictive for cardiovascular outcomes in patients with chronic kidney disease although none is used in the routine diagnostics so far. These marker candidates are involved in processes of inflammation, oxidative stress, or vascular calcification among others.

Several proteins have been identified as molecular biomarker candidates of kidney damage. The clinical significance of these heterogeneous biomarkers is rather difficult to compare due to the variety of clinical settings in which they have been tested such as AKI, diabetic- and non-diabetic CKD, polycystic kidney disease, and dysfunction of kidney grafts. However, their predictive power for progressive decline of kidney function has not been tested in all cases. Moreover, the expression of some of these markers is not kidney specific or restricted to the kidney, and therefore their levels can be influenced by certain non-renal pathologies such as cardiovascular disease, diet, or concomitant medication.

Rudnicki et al (Nephron Exp Nephrol 2004; 97:e86-e95) describe gene expression analysis of a human kidney cell line using cDNA microarrays, and a correlation between microarray and qRT-PCR results.

Rudnicki et al (Kidney International 2007, 71, 325-335) disclose the gene expression profiles of human proximal tubular epithelial cells in proteinuric nephropathies. 168 different genes have been characterized.

Perco et al (European Journal of Clinical Investigation (2006) 36, 753-763) describe protein biomarkers associated with acute renal failure and chronic kidney disease.

Biomarkers indicative for progressive disease are described in PCT/EP2008/068083. Such biomarkers are selected from the group consisting of IL1RN, ISG15, LIFR, C6 and IL32.

Versican is described as an AKI risk factor by PCT/EP2009/054439.

WO2007/096142A2 describes vascular tumor markers, among them versican, and a method for identifying diseases associated with neovascularisation.

Stokes et al (Kidney International 59(2) 532-542, 2001) describe a pathogenic role for versican in crescentic glomerulonephritis (CGN). Renal tissues from CGN patients are immunohistochemically examined for versican, using rabbit polyclonal antibody directed to human versican (VC-E).

WO2009/061368 describes inhibition of versican and antibodies against versican.

Dours-Zimmermann et al (The Journal of Biological Chemistry 269(52) 32992-32998, 1994) disclose the determination of isoforms V0 and V1 in a non-differentiated way, using RT-PCR and immunoblot detection.

Cattaruzza et al (Journal of Biological Chemistry 277(49) 47626-47635, 2002) have carried out a molecular mapping of distributions of PG-M/ versican isoforms V0-V3 in human tissues and investigated how the expression of these isoforms is regulated in endothelial cells in vitro.

Arslan et al (British Journal of Cancer 96(10) 1560-1568, 2007) describe the increased expression of certain versican isoforms in the extracellular matrix, which plays a role in tumor cell growth, adhesion and migration.

WO91/08230 describes antibodies against the NH2-terminal domain or glycosaminoglycan attachment domain of versican.

It is a goal of the present invention to provide a universal marker specifically indicative for renal disorders.

The object is solved by the method according to the invention, which provides for the in vitro determination of the risk of renal disorders in a patient, by measuring a VCAN parameter or a parameter, which is related to VCAN, characterized in that at least one of the isoforms V0 and V1 are specifically determined in a sample of said patient and compared to a reference level. The term "risk of renal disorders" include any kind of renal disorders, the risk of developing renal disorders or the risk of a progressive renal disorder. Determining the risk of renal disorders shall mean the risk assessment as well as determining renal disease, including its diagnosis, prognosis, progression, monitoring and influence of test compounds or therapeutics on such disease.

The term "specific" determination or "specifically" determining with respect to the method according to the invention refers to a reaction of a reagent that is determinative of the versican isoform of interest in a population of molecules comprising the versican isoform of interest and at least one further versican isoform. Thus, under designated assay conditions, the reagent binds to its particular target isoform and does not bind in a significant amount to another isoform or other molecules present in a sample. The specific determination in particular means that the readout is selective in terms of the individual target identity, thus differentiating from other, similar targets, such as other isoforms. The selective determination is usually achieved, if the target recognition is is at least 3 fold different, preferably at least 5 fold different, preferably at least 10 fold different, preferably the difference is at least 100 fold, and more preferred a least 1000 fold.

The preferred method according to the invention relates to the specific determination of both isoforms, i.e. the determination of both isoforms on an individual basis, in the same or the same type of sample.

It was surprisingly found that employing the inventive isoforms of versican a specific risk determination of renal disorders in general is feasible. Preferably said renal disorders are selected from acute, diabetic- and non-diabetic chronic, polycystic, proteinuric or progressive kidney disease, dysfunction of kidney grafts, and associated increased morbidity or mortality from cardiovascular disease and bone metabolism disorders. The method according to the invention would, however, preferably exclude the determinion of renal cancer or tumors.

In particular, by the method according to the invention a renal disease is determined, such as disorders selected from IgA nephropathy, non IgA mesangioproliferative glomerulonephritis, membranoproliferative glomerulonephritis, any postinfectious glomerulonephritis, focal-segmental glomerulosclerosis, minimal change disease, membranous nephropathy, lupus nephritis of any kind, vasculitides with renal involvement of any kind, any other systemic disease leading to renal disease including but not being limited to diabetes mellitus, hypertension or amyloidosis, any hereditary renal disease, any interstitial nephritis and renal transplant failure.

In a preferred method according to the invention the amount of said parameter is increased at least 1.5 times the reference value of subjects not at risk of the renal disorder.

The preferred method comprises sampling from the patient's tissue or body fluid, such as to provide a sample, which is a tissue, blood, serum, plasma or urine sample. In particular, the sample preferably used is a kidney biopsy sample.

The determination method preferably comprises the determination of the VCAN expression, either one of the inventive isoforms or both. The VCAN expression is preferably determined as VCAN nucleic acid and/or protein expression.

A preferred method according to the invention relates to the determination of a respective parameter by microarray hybridization with specific probes or by PCR.

In a preferred method the inventive parameter is tested in combination with a further kidney risk factor (KRF) or senescence parameter. Preferably combined KRF are markers selected from the group consisting of IL1RN, ISG15, LIFR, C6, IL32, NRP1, CCL2, CCL19, COL3A1 and GZMM. Other combinations of any of the inventive versican isoforms with each other or any other relevant biomarker associated with renal disorders and related conditions would be feasible.

Preferably combined senescence parameters are selected from the group consisting of chronological age, telomere length, CDKN2A and CDKN1A. Other senescence parameters commonly used to determine a correlation with chronological age may be employed as well, such as those, which are either regulators of p53, associated with DNA repair, cell cycle control, telomere binding and cell surface remodelling. Exemplary senescence associated genes are selected from the group consisting of Sirtiuns 1-8, XRCC5, G22P1, hPOT 1, Collagenase, TANK 1,2, TRF 1,2 and WRN.

According to the invention there is preferably provided a method for the diagnosis or prognosis of progressive proteinuric kidney disease, renal disease in a patient at risk of disease progression or kidney failure.

A specific aspect of the invention refers to a set of reagents and the use of such set for determining the risk of renal disorders, comprising

- a reagent specifically binding to VCAN V0 polypeptide, and
- a reagent specifically binding to VCAN V1 polypeptide.

In particular, the reagents differentiate between VCAN0 and VCAN1 polypeptides. Either a mixture of the reagents or a set of single components may be provided. The set according to the invention preferably employs reagents, which are antibodies or antibody fragments, preferably monoclonal antibodies specifically recognizing one of the inventive isoforms. Preferably reagents as used in a set according to the invention are used together with detection means, such as a label. Preferred reagents are labelled.

Therefore, the present invention provides a method of determining renal disorders, which is particularly important for determining a progressive disease, e.g. the risk of disease conditions terminally associated with end-stage renal failure. A method for diagnosing a progressive disease and/or assessing long term prognosis of a disease would provide for qualifying high risk patients early on, even before the diagnosis of a chronic disease.

It has been surprisingly found out that the versican V0 and V1 isoforms or splice variants are specifically determinative of high risk patients. Unexpectedly, the expression of the individual isoforms V0 and V1 turned out to be significantly higher in patients with a progressive clinical course of disease. Other isoforms like V2 and V3 did not correlate with renal disorders, such as progressive disease. For the inventive method one of these markers or associated parameters can be detected, which relate to the specific markers with a high correlation.

Versican (VCAN - UniGene: Hs.643801, Hs.715773, GeneID: 1462, GenBank: AA056022/AA056070) is a major extracellular chondroitin sulfate proteoglycan also known as Chondroitin sulfate proteoglycan core protein 2 (CSPG-2), PG-M, or Chondroitin sulfate proteoglycan 2.

VCAN V0, also called VCAN0, is a specific isoform, the transcript variant 1, which corresponds to the longest isoform. The protein sequence is retrieved from the International Protein Index (IPI), a database hosted by the European Bioinformatics Institute (EBI) http://www.ebi.ac.uk/IPI/IPIhelp.html. The sequence of isoform V0 of versican core protein is provided as SEQ ID No: 1.

The VCAN0 mRNA sequence is retrieved from the NCBI nucleotide database. http://www.ncbi.nlm.nih.gov/. The sequence is listed in SEQ ID No. 2

VCAN V1, also called VCAN1, has a shorter sequence than VCAN0. The protein sequence is retrieved from the International Protein Index (IPI), a database hosted by the European Bioinformatics Institute (EBI) http://www.ebi.ac.uk/IPI/IPIhelp.html. The sequence of isoform V1 of versican core protein is provided as SEQ ID No: 3.

The term "VCAN0 and/or VCAN1" as used herein shall refer to markers, including but not limited to respective polypeptides and nucleotide sequences, such as native-sequence polypeptides, chimeric polypeptides, a derivative, an essential part of the splice variants, and precursors thereof, and modified forms of the polypeptides and derivatives, or nucleic acids encoding such polypeptides, which may be included in a biological sample, are referred to herein as inventive isoforms or inventive markers.

Increased expression of the hyaluronan-binding proteoglycan versican was found to be associated with (i) age, (ii) serum creatinine at time of biopsy in diabetic nephropathy, (iii) progressive decline of renal function in proteinuric nephropathies and (iv) acute tubular injury, tubular atrophy and interstitial fibrosis in zero-hour kidney transplant biopsies. When the expression of VCAN was evaluated, it was surprisingly found that the isoforms V0 and V1, but not V2 and V3, were appropriate markers to determine progressive disease. By an exemplary method according to the invention it was found that the expression of the isoforms V0 and V1 was significantly higher in patients with progressive disease (V0: 3.7 fold, $p = 0.0025$; V1: 2.1 fold, $p=0.014$). The isoform V2 was not expressed in these samples, and no differences of the expression of the isoform V3 between stable and progressive subjects was found. In an extended study these results have been confirmed. To evaluate which cells in the kidney might contribute to VCAN expression, the basal expression of all VCAN isoforms was determined in vitro. VCAN isoforms V0 and V1 were highly expressed in various epithelial tubule cell lines and in skin fibroblasts, but to a much lesser extent in foreskin fibroblasts, prostate epithelial cells, smooth muscle cells and colon carcinoma cells. Versican has also been determined by immunohistochemistry in human kidney biopsies. Versican mRNA was determined in a mouse model of glomerulonephritis. The differentiation of the versican isoforms according to the inventive method will provide for the improved determination of renal disorders. The in vitro results particularly suggested a cell specific and an organ specific expression of VCAN V0 and V1 isoforms in the kidney.

As a read out, the amount of parameters in a sample to determine the inventive VCAN markers may be measured and correlated to the risk of said patients, which can be low, medium or high, or else prediction rules established in order to discriminate between the binary outcome stable or progressive disease. For example, the ability of a prediction rule can be assessed by calculating the area under the ROC curve (AUC) using the Sommer's D statistic. The relation between the area under the ROC and Sommer's D is the following:

$$AUC= (1+\text{Sommer's D})/2.$$

It is preferred to employ a marker according to the invention either as single predictor of progression with an AUC value of at least 0.5, preferably at least 0.6, more preferred 0.7, 0.8 or even at least 0.9. Preferred marker combinations reach AUC values of at least 0.6, preferably at least 0.7, 0.8 or even at least 0.9, up to 1.0.

With reference to a healthy patient or a stable disease patient, the preferred method according to the invention qualifies a significant risk when an increase of single parameters by at least 50%, preferably at least 60%, more preferred at least 70%, more preferably at least 80%, more preferably at least 100% is determined.

The high risk progressive nature of the disease is preferably indicated, if the amount of a marker or the combination of markers is increased at least 1.5 times the reference value of subjects not suffering from the progressive disease, preferably being healthy subjects or subjects suffering from a chronic non-progressive disease.

In special embodiments the amount of VCAN0 or VCAN1 is at least 1.5, preferably at least 1.6, at least 1.8, at least 2, at least 3, at least 4, at least 5, at least 6, or at least 8 times the reference value, in particular as determined by PCR with either PPIA or GAPDH as endogenous controls or as determined by microarray analysis.

If more than one marker is detected, the comparison is made to each single reference value for each marker in the non-progressive disease or healthy reference itself.

The inventive method can distinguish if a chronic disease is stable, i.e. the symptoms do not significantly increase over a period of about at least or up to four, six, eight, ten months, one, two or three years after the sample was obtained, or is a progressive disease, i.e. the condition of the subject will increasingly suffer, e.g. over the same time span.

Patients at risk of a renal progressive disease have an increased risk of gradual worsening of renal disease.

The National Kidney Foundation's Kidney Disease Outcomes Quality Initiative (NKF KDOQI) classified chronic kidney disease (CKD) into five stages with stage five indicating terminal kidney failure. Stage 1 patients have kidney damage with normal glomerular filtration rate (GFR) values above 90ml/min/1.73m2. Patients in stage two have slightly decreased GFR values between 60 and 89 ml/min/1.73m2. Stage three patients have moderately decreased GFR values between 30 and 59 ml/min/1.73m2. Patients in stage four experience severely decreased GFR values between 15-29 ml/min/1.73m2. Kidney failure, also defined as end-stage renal disease, is reached in stage five when patients have GFR values lower than 15 ml/min/1.73m2. End-stage renal disease is followed by renal replacement therapy with the treatment options dialysis or organ transplantation.

If the risk of end-stage renal failure is high, the disease stages will be passed very quickly, which would result in the need for kidney dialysis and transplantation. To delay the terminal phase of renal disease a patient which was diagnosed as having an increased risk of disease progression would receive the appropriate medication early on employing aggressive treatment regimens.

The risk of a patient to suffer from kidney or renal disease progression may be diagnosed at an early stage of disease, even before a chronic disease has been diagnosed. On the other hand a prognosis is provided, which would quantify the fast progression of the disease in a patient already suffering from chronic renal disease.

Thus, the inventive method can include the step of obtaining the sample from a patient potentially suffering from a progressive renal disease, where a chronic renal disease may already have been diagnosed or not. The method according to the invention is preferably employed with a kidney biopsy sample, such as wedge or needle sample, or else from tubular cells, and also by detecting the markers in serum, blood, plasma and urine by comparing reference values of standard values or from healthy subjects.

The term "patients" herein includes subjects suffering from or at risk of renal disorders, but also healthy subjects. The subject can, e.g., be any mammal, in particular a human, but also selected from mouse, rat, hamster, cat, dog, horse, cow, pig, etc. The inventive method can also include the step of obtaining the sample from a patient at risk for developing acute kidney injury, e.g. before contrast medium administration in the course of angiography.

The invention also provides a method of assessing whether a patient is at risk of a renal disorder, comprising comparing:

(a) levels of the V0 and/or V1 isoform(s) in a sample from said patient, and

(b) normal levels of said isoform(s) in samples of the same type obtained from control patients, wherein altered levels of the isoform(s) relative to the corresponding normal levels is an indication that the patient has a risk of renal disorder, e.g. a predisposition to kidney disease, such as AKI or disease progression, in particular where detection of a level of an isoform that differs significantly from the standard indicates acute kidney disease or onset of kidney disease or increased risk for developing ARF or disease progression. A significant difference between the levels of an inventive isoform in a patient and the normal levels is an indication that the patient has a risk of kidney disease or a predisposition to kidney disease.

It is explicitly understood that the method according to the invention is carried out in vitro, including ex vivo settings.

The inventive markers can be detected in any sample of a subject comprising said markers e.g. where an expression of an inventive isoform is determined either as polynucleotide, e.g. as mRNA, or expressed polypeptide or protein. The comparison with the reference should be of the same sample type. The comparison with the reference value should be of the same sample type. In particular, the sample can be tissue, e.g. of a biopsy, blood, serum, plasma or a urine sample.

Reference values for the inventive isoforms are preferably obtained from a control group of patients or subjects with normal expression of said isoform, or an isoform expression, that is afflicted with kidney stress conditions, such as septic, cancer or diabetic patients, without proteinuremia or AKI, which represents the appropriate reference patient group. In a particular aspect, the control comprises material derived from a pool of samples from normal patients.

The term "detect" or "detecting" includes assaying, imaging or otherwise establishing the presence or absence of the target versican isoform encoding the markers, subunits thereof, or combinations of reagent bound targets, and the like, or assaying for, imaging, ascertaining, establishing, or otherwise determining one or more factual characteristics of kidney disease or similar conditions. The term encompasses diagnostic, prognostic, and monitoring applications for an inventive versican isoform.

In preferred embodiments, determining the amount of the inventive marker or any combination thereof comprises determining the expression of the marker(s), preferably by determining the mRNA concentration of the marker(s). To this extent, mRNA of the sample can be isolated, if necessary after adequate sample preparation steps, e.g. tissue homogenisation, and hybridized with marker specific probes, in particular on a microarray platform with or without amplification, or primers for PCR-based detection methods, e.g. PCR extension labelling with probes specific for a portion of the marker mRNA. In preferred embodiments the marker(s) or a combination thereof is (are) determined by microarray hybridization with VCAN0 and/or VCAN1 specific probes, or by PCR.

Differential expression of the polynucleotides is preferably determined by microarray, hybridization or by amplification of the extracted polynucleotides. The invention contemplates a gene expression profile comprising one or both of the inventive markers. This profile provides a highly sensitive and specific test with both high positive and negative predictive values permitting diagnosis and prediction of the patient’s risk of developing disease.

For example, the invention provides a method for determining the risk of renal disorders in a patient comprising

(a) contacting a sample obtained from said patient with oligonucleotides that specifically hybridize to the V0 and/or V1 isoform(s), and
(b) detecting in the sample a level of polynucleotides that hybridize to the isoform(s) relative to a predetermined cut-off value, and therefrom determining the risk of renal disorders in the subject.

Within certain preferred embodiments, the amount of polynucleotides that are mRNA are detected via polymerase chain reaction using, for example, oligonucleotide primers that hybridize to an inventive isoform, or complements of such polynucleotides. Within other embodiments, the amount of mRNA is detected using a hybridization technique, employing oligonucleotide probes that hybridize to an inventive isoform, or complements thereof. When using mRNA detection, the method may be carried out by combining isolated mRNA with reagents to convert to cDNA according to standard methods and analyzing the products to detect the presence of the isoform in the sample.
In particular aspects of the invention, the methods described herein utilize one or both inventive markers placed on a microarray so that the expression status of each of the markers is assessed simultaneously. In an embodiment, the invention provides a microarray comprising a defined set of marker genes, whose expression is significantly altered by a risk of renal disorders. The invention further relates to the use of the microarray as a prognostic tool to predict kidney disease.
In further embodiments the amount of a marker or any combination thereof is determined by the polypeptide or protein concentration of the marker(s), e.g. with marker specific ligands, such as antibodies or specific binding partners. The binding event can, e.g., be detected by competitive or non-competitive methods, including the use of labelled ligand or marker specific moieties, e.g. antibodies, or labelled competitive moieties, including a labelled marker standard, which compete with marker proteins for the binding event. If the marker specific ligand is capable of forming a complex with the marker, the complex formation indicates expression of the markers in the sample.
In particular, the invention relates to a method for diagnosing and/or monitoring renal disorders in a patient by quantitating the V0 and/or V1 isoform(s) in a sample from the subject comprising

(a) reacting the sample with one or more binding agents specific for the isoform(s), e.g. an antibody that is directly or indirectly labelled with a detectable substance, and
(b) detecting the detectable substance.

VCAN isoform levels can be determined by constructing an antibody microarray, in which binding sites comprise immobilized, preferably monoclonal antibodies specific to a marker. The invention also relates to kits for carrying out the methods of the invention.
The invention further contemplates the methods, compositions, and kits described herein using additional markers associated with kidney disease. The methods described herein may be modified by including reagents to detect the additional markers, or polynucleotides for the markers.
Appropriate probes, specific antibodies or methods for determining the biomarkers are known in the art, and have been used for different purposes.
In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers.
The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.
Antibodies or other polypeptides may be immobilized onto a variety of solid supports for use in assays. Solid phases that may be used to immobilize specific binding members include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot.

Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non- specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In exemplary embodiments, the analyte is measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Marker specific moieties are substances which can bind to or detect at least one of the markers for a detection method described above and are in particular marker nucleotide sequence detecting tools or marker protein specific antibodies, including antibody fragments, such as Fab, F(ab), F(ab)', Fv, scFv, or single chain antibodies. The marker specific moieties can also be selected from marker nucleotide sequence specific oligonucleotides, which specifically bind to a portion of the marker sequences, e.g. mRNA or cDNA, or are complementary to such a portion in the sense or complementary anti-sense, like cDNA complementary strand, orientation.

For easy detection the moieties are preferably labelled, such as by optical, including fluorescence, and radioactive labels.

The inventive prognosis method can predict whether a patient is at risk of developing acute kidney injury. The higher the fold increase, the higher is the patient's risk of AKI. An elevated level of an inventive isoform indicates, for example, special treatment of the patient, using appropriate medication or contrast media. The method of the invention can thus be used to evaluate a patient before, during, and after medical treatment.

Likewise, the inventive isoform level can be compared to a cut-off concentration and the kidney disease development potential is determined from the comparison; wherein concentrations of the versican isoform above the reference concentrations are predictive of, e.g., correlate with, kidney disease development in the patient.

Thus, the preferred method according to the invention comprises the step of comparing the KRF level with a predetermined standard or cut-off value, which is preferably at least 50% higher than the standard, more preferred at least 60% or 70% higher, but can also be at least 100% higher.

In aspects of the methods of the invention, the methods are non- or minimally invasive for renal disorders predisposition testing, which in turn allow for diagnosis of a variety of conditions or diseases, e.g. associated with acute kidney disease. In particular, the invention provides a non-invasive non-surgical method for detection, diagnosis, monitoring, or prediction of acute kidney disease or onset of kidney disease in a patient comprising: obtaining a sample of blood, plasma, serum, urine or saliva or a tissue sample from the patient; subjecting the sample to a procedure to detect one or both of the inventive isoforms by comparing the levels of the isoform to the levels of the isoform obtained from a control.

The invention also contemplates a method of assessing the potential of a test compound to contribute to kidney disease or onset of kidney disease comprising:

(a) maintaining separate aliquots of a sample from a patient in the presence and absence of the test compound, and

(b) comparing the levels of the V0 and/ or V1 isoform(s) in each of the aliquots.

This is particularly useful in monitoring the versican isoform level in clinical trials. A significant difference between the levels of an inventive isoform in an aliquot maintained in the presence of or exposed to the test compound relative to the aliquot maintained in the absence of the test compound, indicates that the test compound potentially contributes to kidney disease or onset of kidney disease.

Likewise, the invention can be employed to determine the effect of an environmental factor on kidney disease comprising comparing one or both of the inventive isoforms associated with kidney disease or onset of kidney disease in the presence and absence of the environmental factor.

In a further aspect the present invention provides a set that contains or consists of at least two different reagents or marker specific moieties, to specifically determine both of the inventive VCAN variants on an individual basis. Besides, further markers may be determined in the same sample for the same or a different purpose.

Marker specific moieties used as preferred reagents in such a set according to the invention are substances which can bind to or detect at least one of the markers for a detection method described above and are in particular marker protein specific antibodies or antibody fragments, such as Fab, F(ab)2, F(ab)', Fv, scFv, or single chain antibodies. The marker specific moieties can also be selected from marker nucleotide sequence specific oligonucleotides, which specifically bind to a portion of the marker sequences For easy detection the moieties are preferably directly or indirectly labelled, such as by optical, including fluorescence, and radioactive labels.

Figures:

Figure 1. Correlation of versican isoform V0 RNA levels with eGFR at time of biopsy (A) and with eGFR at latest follow up (B), and of V1 RNA levels with eGFR at time of biopsy (C) and with eGFR at latest follow up (D). eGFR estimated glomerular filtration rate in ml/min/1.73m2

Figure 2. Versican isoform expression in stable and progressive kidney diseases.

Figure 3. Expression of versican mRNA in vitro. The basal expression of versican isoforms was measured in various renal and non-renal cell lines. K2 primary proximal tubule cells, HK2 and hTERT-RPTC immortalized renal proximal tubule cells, HF primary skin fibroblasts, VHF primary foreskin fibroblasts, SMC primary smooth muscle cells, EP immortalized prostate epithelial cells, CACO2 colon carcinoma cells, LLC-PK1 pig renal tubule cells, kidney: whole kidney tissue. The expression values are shown as ratio to PPIA.

Figure 4. VCAN expression in a mouse model of glomerulonephritis.

Figure 5. Versican protein expression in renal disease.

Versican protein expression was detected in representative stable and progressive subjects (arrows). Versican protein was expressed both in the glomerular (A) and in the tubulointerstitial (B) compartment, however, expression was more prominent at the tubular basal membrane (B) and in the interstitiu (C). Furthermore, versican was also detected in the media of renal cortical blood vessels (D).

The present invention is further illustrated by the following figures and examples without being limited thereto.

Examples:

The incidence and prevalence of chronic kidney disease (CKD) is increasing worldwide and has been predicted to soon reach epidemic proportions. Chronic kidney diseases is caused by primary renal diseases such as IgA nephropathy (IgAN), minimal change disease (MCD), focal-segmental glomerulosclerosis (FSGS), membranous nephropathy (MN) and membranoproliferative glomerulonephritis (MPGN), as well as by systemic diseases (e.g. systemic lupus erythematodes, diabetes mellitus type 1 and type 2, or hypertension), which can also lead to deterioration of kidney function. In a proportion of these patients CKD progresses to end-stage renal disease (ESRD) which requires renal replacement therapy such as dialysis and kidney transplantation. These therapies represent a major challenge for healthcare systems. But even slight impairment of kidney function - far from ESRD - correlate with serious health consequences such as increased cardiovascular morbidity and mortality (e.g. myocardial infarction, sudden cardiac death, peripheral arterial disease), increased risk of pathological bone fractures due to renal osteodystrophy, and consequently with reduced quality of life.

To date only few general risk factors for the progression of renal failure have been firmly established. It is known that elevated serum creatinine at time of biopsy, hypertension, and the degree of proteinuria (typically > 500 - 1000 mg/day) correlate with an unfavourable prognosis in various glomerulopathies. Although these clinical findings are of stronger predictive value, certain histopathological changes on kidney biopsies have also been associated with increased risk of progression. The degree of tubular atrophy and interstitial fibrosis is a better predictor of long-term renal survival than the extent of glomerular damage in almost all glomerular renal diseases including IgA nephropathy (IgAN), membranous nephropathy (MN), membranoproliferative glomerulonephritis (MPGN) and lupus nephritis (LN).

Herein the VCAN isoforms V0 and V1 were found to be novel biomarkers for adverse outcome in CKD.

Materials and Methods

**Isoforms of Versican.** Five isoforms of versican (GeneID 1462) are listed in the International Protein Index (IPI) as given in the table below. Four of these isoforms (V0, V1, V2 and V3) are confirmed splice variants of the versican gene. The isoform Vint has been proposed as another isoform, which largely resembles isoform V0 and differs solely by a deletion/insertion in the carboxyterminal end of the RNA.

Table 1: VCAN isoforms as listed in the International Protein Index

| IPI | Accession | Description | SeqLength |
|---|---|---|---|
| IPI:IPI00009802.1 | IPI00009802 | Isoform V0 of versican core protein | 3396 |
| IPI:IPI00215628.1 | IP100215628 | Isoform V1 of versican core protein | 2409 |
| IPI:IP100215629.1 | IPI00215629 | Isoform V2 of versican core protein | 1642 |
| IPI:IP100215630.1 | IPI00215630 | Isoform V3 of versican core protein | 655 |
| IPI:IPI00215631.1 | IPI00215631 | Isoform VINT of versican core protein | 3370 |

**Example 1: Patients and kidney biopsies.**

In a first setting, we used 37 kidney biopsies obtained from patients with proteinuric renal diseases during their routine diagnostic workup for which we had complete clinical follow-up data (Table 2): diabetic nephropathy n = 2, hypertensive nephropathy n = 2, IgA nephropathy n = 11, minimal change disease n = 8, membranous nephropathy n = 7, primary focal-segmental glomerulonephritis n = 6, unknown n = 1). The median follow-up time was 25 months (2 - 80). Based upon the estimated glomerular filtration rate (eGFR), which was calculated using the modified MDRD formula, patients were divided into a stable and a progressive cohort: Patients were defined stable when eGFR was > 60 ml/min/1.73m$^2$ at both timepoints, or when eGFR was < 60 ml/min/1.73m$^2$ at either timepoint and no decline in eGFR over time was observed. Patients were defined as progressive when eGFR was > 60 ml/min/1.73m$^2$ at time of biopsy and < 60 ml/min/1.73m$^2$ during follow-up, or when eGFR < 60 ml/min/1.73m$^2$ at both timepoints and delta eGFR was less than -1 ml/min/1.73m$^2$, or when they reached end-stage renal disease. Tubular atrophy and interstitial fibrosis (TAIF) were scored by an independent pathologist following a semiquantitative grading system on haematoxylin/eosin and periodic-acid-Schiff- or Pearse-stained sections: none, mild (0 - 10 %), moderate (11-30 %), severe (> 30 %). The use of surplus material from routine biopsies for gene expression profiling has been accredited by the Institutional Review Board of the Medical University of Innsbruck.

**RNA isolation and real-time PCR.** Total RNA of whole kidney cryosections was isolated using the RNeasy® Micro Kit (Qiagen, Valencia CA). RNA was reverse transcribed into cDNA with the High Capacity cDNA reverse Transcription kit (Applied Biosystems, Foster City CA) in a 50 μl reaction according to the manufacturer's instructions. Preamplification was performed using TaqMan® Gene Expression Assays (*vide infra*) and the TaqMan® PreAmp Master Mix. Briefly, equal volumes of 20x TaqMan Gene Expression Assays were pooled and diluted to 0.2x with TE buffer. A 50 μl reaction containing 12.5 μl pooled assay mix, 25 μl TaqMan Preamp Master Mix and 5ng of cDNA was prepared per sample and incubated in a thermocycler for 10 min at 95°C followed by 10 cycles of 95°C for 15 seconds and 60°C for 4 minutes. Samples were then immediately cooled and diluted to 250 μl with TE buffer. All gene expression assays used had been previously tested to ensure uniform preamplification as recommended by the manufacturer.

The preamplified cDNA was analysed on the 7500 Fast Real-Time PCR System (Applied Biosystems) using the following inventoried TaqMan® Gene Expression Assays: PPIA (cyclophilin A; Hs99999904_m1), VCAN0 (Hs01007944_m1), VCAN1 (Hs01007937_m1), VCAN2 (Hs01007943_m1) and VCAN3 (Hs01007941_m1). Information about the alignments of the primers and the probes are publicly available at the manufacturers homepage www.appliedbiosystems.com using the TaqMan® Gene Expression Assay numbers listed above. Each reaction contained 10 μl of Gene Expression Master Mix, 1μl of TaqMan Gene Expression Assay, 5 μl preamplified cDNA and 4 μl $H_2O$. Reactions were prepared in duplicate for each sample and incubated at 50°C for 2 minutes, 95°C for 10 minutes followed by 40 cycles of 95°C for 15 seconds and 60°C for 1 minute. The relative amounts of transcripts for each gene were normalised to the reference gene PPIA as follows: deltaC$_T$ = C$_T$ (gene of interest) - C$_T$ (PPIA). The deltaC$_T$ was linearized according to the formula $2^{-dCT}$ to determine the relative expression of each gene of interest.

**Cell culture.** For versican mRNA expression studies we used several cell lines of epithelial or mesenchymal origin: Renal proximal tubule cells derived from human (HK2) and pig (LLC-PK1), colon carcinoma cells (CACO-2), as well as human endothelial cells (EA.hy926) were purchased from American Type Culture Collection (ATCC). Primary proximal tubule cells (K2) were provided by Dr. C. Koppelstaetter (Department of Nephrology, Innsbruck Medical University, Austria). Immortalized prostate epithelial cells (EP156T, EP153T), primary smooth muscle cells (SMC), primary foreskin fibroblasts (VHF), and primary skin fibroblasts (HF) were obtained from Dr. Iris E. Eder at the Department of Urology from the Innsbruck Medical University. Real-time PCR of the versican isoforms was performed as described above, but the RNA was not pre-amplified. Ct values of versican and PPIA as assessed by ABI sequence detection software (version 1.3) were used to calculate the deltaCt using Microsoft Excel. Values are shown as ratio to the housekeeper PPIA (2 exp deltaCt).

Results I:

**Identification of versican expression as biomarker of progressive renal disease.** We evaluated the expression of versican isoforms V0, V1, V2 and V3 in an independent cohort of 37 patients with various proteinuric kidney diseases (Table 2). The expression of versican isoforms V0 and V1 showed a significant negative correlation with eGFR at time of biopsy and with eGFR at time of follow up (Figure 1). We did not detect any expression of the isoform V2 in these samples. The versican isoform V3 showed a weak downregulation in subjects with lower eGFR, which was statistically significant (p=0.011) but clinically irrelevant (Figure 2). Patients were classified as stable or progressive according to changes in eGFR during a median follow-up time of 25 months (2 - 80 months). As shown in Figure 2, the expression of the isoforms V0 and V1 was significantly higher in progressive disease (V0: 3.7 fold, p = 0.0025; V1: 2.1 fold, p=0.014). The V2 isoform was not expressed in these samples. The versican isoform V3 was downregulated in progressive patients by 2 %. No significant correlation of versican expression to proteinuria, the degree of tubular atrophy and interstitial fibrosis nor the histological diagnosis could be detected. Linear regression analysis was performed for the different VCAN isoforms using the estimated GFR at follow up time as dependent variable. The expression of VCAN isoform 0 was negatively correlated with the estimated GFR (Pearson R = -0.54) and was the single most predictive VCAN isoforms explaining 27.7% (p-value < 0.001) of the variability of the estimated GFR. The VCAN isoforms 1 explained 20.8% (p-value = 0.002) of estimated GFR values at time of follow up. These results suggest a better predictive value of the versican isoforms V0 and V1 for progression of kidney disease, compared to established riskfactors such as degree of tubular atrophy and interstitial fibrosis and/or proteinuria.

**Table 2. Patients included in the analysis of VCAN expression.** HD hemodialysis, NTX kidney transplantation. For abbreviation of the histological diagnosis see *Materials and Methods* section.

| Subject number | sex | Age (years) | eGFR biopsy (ml/min/m$^2$) | Proteinuria biopsy (g/d) | follow up time (months) | ESRD | eGFR follow-up (ml/min/m$^2$) | Proteinuria follow-up (g/d) | delta GFR ml/min/year | Histological diagnosis |
|---|---|---|---|---|---|---|---|---|---|---|
| ***Stable disease*** | | | | | | | | | | |
| NC07 | m | 29 | 75 | 2,0 | 80 | - | 97 | 0,4 | 3,31 | IGAN |
| NC10 | m | 53 | 103 | 1,8 | 24 | - | 117 | 1,6 | 7,00 | IGAN |
| NC11 | m | 44 | 88 | 0,7 | 25 | - | 93 | 0,2 | 2,67 | IGAN |
| NC13 | m | 26 | 101 | 0,6 | 34 | - | 91 | 0,4 | -3,48 | IGAN |
| NC16 | m | 31 | 77 | 7,0 | 24 | - | 92 | 0,1 | 7,62 | MCN |
| NC17 | f | 56 | 109 | 17,0 | 30 | - | 134 | 0,0 | 9,72 | MCN |
| NC18 | m | 41 | 77 | 1,3 | 27 | - | 78 | 0,6 | 0,36 | MCN |
| NC19 | m | 69 | 57 | 8,0 | 24 | - | 61 | 0,2 | 2,18 | MCN |
| NC23 | m | 71 | 63 | 3,4 | 24 | - | 65 | 0,2 | 0,76 | MN |
| NC27 | f | 26 | 115 | 2,9 | 24 | - | 132 | 0,1 | 8,36 | pFSGS |
| NC43 | m | 31 | 55 | 4,4 | 25 | - | 55 | 2,2 | -0,15 | pFSGS |
| NC56 | f | 42 | 85 | 1,8 | 26 | - | 77 | 5,4 | -3,52 | pFSGS |
| NC70 | f | 31 | 113 | 5,8 | 25 | - | 85 | 3,7 | -13,53 | MCN |
| NC72 | m | 53 | 87 | 8,7 | 25 | - | 77 | 1,9 | -4,78 | MN |
| NC76 | f | 53 | 43 | 9,6 | 25 | - | 58 | 0,0 | 7,48 | MCN |
| NC81 | m | 20 | 136 | 2,2 | 12 | - | 112 | 0,1 | -23,06 | MCN |
| NC82 | f | 54 | 81 | 11,3 | 23 | - | 86 | 0,9 | 2,93 | MCN |
| ***Progressive disease*** | | | | | | | | | | |
| NC01 | m | 51 | 12 | 7,2 | 5 | HD | 7 | 9,1 | -11,98 | DN |
| NC06 | m | 29 | 15 | 3,2 | 6 | NTX | 6 | 1,4 | -18,01 | IGAN |
| NC14 | f | 24 | 75 | 1,3 | 24 | - | 53 | 0,2 | -11,34 | IGAN |
| NC29 | m | 54 | 70 | 3,3 | 29 | - | 19 | 1,0 | -21,16 | DN |
| NC31 | m | 58 | 26 | 5,1 | 26 | - | 21 | 1,9 | -2,29 | HN |
| NC32 | f | 47 | 54 | 1,7 | 61 | - | 14 | 1,4 | -8,06 | HN |
| NC33 | m | 59 | 34 | 2,9 | 26 | - | 30 | 3,2 | -1,96 | U |
| NC34 | m | 41 | 16 | 3,0 | 2 | HD | 8 | 3,4 | -54,86 | IGAN |
| NC35 | m | 42 | 48 | 0,9 | 34 | - | 41 | 0,3 | -2,44 | IGAN |
| NC37 | m | 48 | 38 | 3,6 | 25 | - | 21 | 2,7 | -7,97 | IGAN |

(continued)

| | | | | | | | | | | |
|---|---|---|---|---|---|---|---|---|---|---|
| ***Progressive disease*** | | | | | | | | | | |
| NC38 | m | 20 | 96 | 1,7 | 41 | - | 14 | 3,3 | -24,20 | IGAN |
| NC39 | f | 63 | 37 | 8,5 | 26 | - | 11 | 6,9 | -12,32 | MN |
| NC42 | f | 20 | 47 | 1,7 | 32 | - | 40 | 0,4 | -2,78 | pFSGS |
| NC44 | m | 43 | 57 | 4,5 | 26 | - | 41 | 5,4 | -7,50 | pFSGS |
| NC48 | m | 35 | 16 | 4,8 | 4 | HD | 10 | 2,0 | -15,42 | IGAN |
| NC50 | m | 71 | 54 | 3,6 | 21 | - | 33 | n.a. | -11,86 | pFSGS |
| NC51 | m | 51 | 100 | 1,3 | 26 | - | 23 | 7,6 | -36,09 | MN |
| NC52 | m | 71 | 68 | 2,5 | 25 | - | 31 | 2,4 | -18,05 | MN |
| NC73 | f | 69 | 79 | 4,8 | 27 | - | 50 | 1,2 | -12,89 | MN |
| NC89 | f | 63 | 154 | 3,0 | 22 | - | 31 | 1,0 | -65,68 | MN |

**Example 2: VCAN mRNA expression in human kidney biopsies Patients and kidney biopsies**

We extended the Results I above and used kidney biopsies obtained from 74 patients with proteinuric renal diseases during their routine diagnostic workup for which we had complete clinical follow-up data (Table 3): diabetic nephropathy n = 3, hypertensive nephropathy n = 6, IgA nephropathy n = 19, minimal change disease n = 9, membranous nephropathy n = 8, focal-segmental glomerulonephritis n = 8, goodpasture syndrome n = 2, interstitial nephritis n = 4, lupus nephritis n = 2, membranoproliferative glomerulonephritis n = 2, ANCA-associated ANCA vasculitis n = 6, rapid-progressive glomerulonephritis n = 1, unknown and other n = 4. The median follow-up time was 25 months (2 - 80). Based upon the estimated glomerular filtration rate (eGFR), which was calculated using the modified MDRD formula, patients were divided into a stable and a progressive cohort: Patients were defined stable when eGFR was > 60 ml/min/1.73m$^2$ at both timepoints, or when eGFR was < 60 ml/min/1.73m$^2$ at either timepoint and the decline in eGFR over time was > -1 ml/min/1.73m$^2$. Patients were defined as progressive when eGFR was > 60 ml/min/1.73m$^2$ at time of biopsy and < 60 ml/min/1.73m$^2$ during follow-up, or when eGFR < 60 ml/min/1.73m$^2$ at both timepoints and delta eGFR was less than -1 ml/min/1.73m$^2$, or when they reached end-stage renal disease. Tubular atrophy and interstitial fibrosis (TAIF) were scored by an independent pathologist following a semiquantitative grading system on haematoxylin/eosin and periodic-acid-Schiff- or Pearse-stained sections: none, mild (1 - 10 %), moderate (11-30 %), severe (> 30 %). The use of surplus material from routine biopsies (i.e. biopsy material, serum and urine) for gene expression profiling has been accredited by the Institutional Review Board of the Medical University of Innsbruck.

**RNA isolation and real-time PCR**

Total RNA of whole kidney cryosections was isolated using the RNeasy® Micro Kit (Qiagen, Valencia CA). RNA was reverse transcribed into cDNA with the High Capacity cDNA reverse Transcription kit (Applied Biosystems, Foster City CA) in a 50 μl reaction according to the manufacturer's instructions. Preamplification was performed using TaqMan® Gene Expression Assays (*vide infra*) and the TaqMan® PreAmp Master Mix. Briefly, equal volumes of 20x TaqMan Gene Expression Assays were pooled and diluted to 0.2x with TE buffer. A 50 μl reaction containing 12.5 ul pooled assay mix, 25 μl TaqMan Preamp Master Mix and 5ng of cDNA was prepared per sample and incubated in a thermocycler for 10 min at 95°C followed by 10 cycles of 95°C for 15 seconds and 60°C for 4 minutes. Samples were then immediately cooled and diluted to 250 ul with TE buffer. All gene expression assays used had been previously tested to ensure uniform preamplification as recommended by the manufacturer.

The preamplified cDNA was analysed on the 7500 Fast Real-Time PCR System (Applied Biosystems) using the following inventoried human TaqMan® Gene Expression Assays: PPIA (cyclophilin A; Hs99999904_m1), VCAN0 (Hs01007944_m1), VCAN1 (Hs01007937_m1), VCAN2 (Hs01007943_m1) and VCAN3 (Hs01007941_m1). For real-time PCR experiments on RNA extracted from mouse tissue we used the following inventoried TaqMan® Gene Expression Assays: 18s (Hs03003631_g1), VCAN (Mm00490179_m1). Each reaction contained 10 μl of Gene Expression Master Mix, 1μl of TaqMan Gene Expression Assay, 5μl preamplified cDNA and 4 μl $H_2O$. Reactions were prepared in duplicate for each sample and incubated at 50°C for 2 minutes, 95°C for 10 minutes followed by 40 cycles of 95°C for 15 seconds and 60°C for 1 minute. The relative amounts of transcripts for each gene were normalised to the reference gene PPIA in human and 18s in mouse samples as follows: $\Delta C_T = C_T$ (gene of interest) - $C_T$ (PPIA). The $\Delta C_T$ was linearized according to the formula $2^{-dCT}$ to determine the relative expression of each gene of interest.

**Identification of versican expression as a biomarker of progressive renal disease**

The expression of versican isoforms V0, V1, V2 and V3 was evaluated in an extended cohort of 74 patients with various proteinuric kidney diseases (Table 3). The expression of versican isoforms V0 and V1 showed a significant negative correlation with eGFR at time of biopsy and with eGFR at time of follow up: V0 vs eGFR biopsy r = -0.314 (p-value = 0.003), V1 vs eGFR biopsy r = -0.303 (p-value = 0.009), V0 vs eGFR follow-up r = -0.371 (p-value = 0.0010) and V1 vs eGFR follow-up r = -0.385 (p-value = 0.0007). We did not detect any expression of the isoform V2 in these samples. The versican isoform V3 did not show any correlation with eGFR. We did not detect any significant correlation of versican isoform expression with gender, age, proteinuria (biopsy and follow-up), histological diagnosis and the degree of tubular atrophy and interstitial fibrosis. The expression levels of the V1 isoform significantly correlated with the degree of interstitial inflammatory infiltrate (Kruskal Wallis test p-value: 0.014).

Patients were classified as stable or progressive according to changes in eGFR during a median follow-up time of 25 months (2 - 80 months). The expression of the isoforms V0 and V1 at time of biopsy was higher in patients with a progressive clinical course of the disease (V0: 1.7 fold, p = 0.02; V1: 1.6 fold, p=0.05). The V2 isoform was not expressed in these samples. The versican isoform V3 did not show any difference in expression between stable and progressive patients.

**Table 3. Patients included in the analysis of VCAN expression.** HD hemodialysis, NTX kidney transplantation. For abbreviation of the histological diagnosis see text.

| Subject number | sex | Age (years) | eGFR (ml/min/m$^2$) | Proteinuria (g/d) | follow up (months) | ESRD? | eGFR (ml/min/m$^2$) | Proteinuria (g/d) | delta GFR ml/min/year | Histological diagnosis |
|---|---|---|---|---|---|---|---|---|---|---|
| **Stable disease** | | | | | | | | | | |
| **NC02** | f | 70 | 29 | 7,2 | 28 | - | 42 | 0,09 | 5,8 | DN |
| **NC04** | m | 46 | 40 | 0,9 | 28 | - | 49 | 0,24 | 4,0 | HN |
| **NC05** | m | 55 | 107 | 0,7 | 39 | - | 107 | unknown | 0,0 | HN |
| **NC07** | m | 29 | 75 | 2,0 | 80 | - | 95 | 0,40 | 3,0 | IGAN |
| **NC08** | f | 41 | 28 | 4,1 | 25 | - | 31 | 1,14 | 1,5 | IGAN |
| **NC10** | m | 53 | 103 | 1,8 | 24 | - | 67 | 1,57 | -18,7 | IGAN |
| **NC11** | m | 44 | 88 | 0,7 | 25 | - | 94 | 0,20 | 2,8 | IGAN |
| **NC12** | m | 33 | 55 | 1,2 | 24 | - | 57 | 0,71 | 0,7 | IGAN |
| **NC13** | m | 26 | 101 | 0,6 | 34 | - | 83 | 0,36 | -6,3 | IGAN |
| **NC14** | f | 24 | 75 | 1,3 | 24 | - | 62 | 0,23 | -6,7 | IGAN |
| **NC16** | m | 31 | 77 | 7,0 | 24 | - | 89 | 0,07 | 6,3 | MCD |
| **NC17** | f | 56 | 81 | 17,0 | 30 | - | 61 | 0,02 | -7,9 | MCD |
| **NC18** | m | 41 | 77 | 1,3 | 27 | - | 72 | 0,62 | -2,5 | MCD |
| **NC19** | m | 69 | 57 | 8,0 | 24 | - | 81 | 0,23 | 11,9 | MCD |
| **NC23** | m | 71 | 63 | 3,4 | 24 | - | 72 | 0,20 | 4,3 | MN |
| **NC24** | f | 71 | 6 | 3,9 | 24 | - | 39 | 0,11 | 16,5 | IN |
| **NC25** | m | 23 | 36 | 0,3 | 25 | - | 44 | 0,92 | 4,1 | IN |
| **NC26** | f | 41 | 84 | 1,3 | 25 | - | 100 | 0,14 | 7,4 | RPGN |
| **NC27** | f | 26 | 115 | 2,9 | 24 | - | 95 | 0,15 | -10,1 | pFSGS |
| **NC50** | m | 71 | 54 | 3,6 | 21 | - | 58 | n.a. | 2,7 | pFSGS |
| **NC53** | m | 49 | 81 | 1,4 | 25 | - | 70 | 11,18 | -5,1 | LN |
| **NC54** | f | 51 | 22 | 3,8 | 24 | - | 35 | 2,90 | 6,6 | LN |
| **NC55** | f | 64 | 28 | 10,3 | 31 | - | 43 | 0,30 | 6,0 | pFSGS |
| **NC56** | f | 42 | 85 | 1,8 | 26 | - | 64 | 5,36 | -9,5 | pFSGS |
| **NC57** | m | 24 | 53 | 0,6 | 24 | - | 77 | 0,04 | 12,2 | IGAN |
| **NC58** | f | 59 | 35 | 3,3 | 25 | - | 47 | 0,53 | 5,4 | MPGN |
| **NC59** | f | 24 | 10 | 0,7 | 25 | - | 71 | 0,72 | 29,7 | Goodpasture |
| **NC60** | m | 29 | 19 | 0,4 | 24 | - | 48 | 0,42 | 14,2 | HN |
| **NC62** | m | 37 | 104 | 1,9 | 27 | - | 92 | 1,96 | -5,0 | IGAN |
| **NC63** | m | 20 | 67 | 10,8 | 28 | - | 103 | 0,62 | 15,4 | Goodpasture |
| **NC64** | m | 81 | 20 | 0,4 | 25 | - | 29 | 0,17 | 4,6 | Vasculitis |

(continued)

| Subject number | sex | Age (years) | eGFR (ml/min/m$^2$) | Proteinuria (g/d) | follow up (months) | ESRD? | eGFR (ml/min/m$^2$) | Proteinuria (g/d) | delta GFR ml/min/year | Histological diagnosis |
|---|---|---|---|---|---|---|---|---|---|---|
| **Stable disease** | | | | | | | | | | |
| **NC65** | f | 53 | 59 | 0,6 | 25 | - | 58 | 0,26 | -0,3 | IGAN |
| **NC66** | f | 72 | 24 | 2,5 | 24 | - | 58 | 0,00 | 16,8 | Vasculitis |
| **NC67** | m | 60 | 49 | 0,1 | 21 | - | 64 | 0,07 | 8,7 | IN |
| **NC68** | m | 37 | 19 | 0,6 | 24 | - | 72 | 0,17 | 26,1 | IGAN/Vasc. |
| **NC69** | m | 49 | 55 | 0,0 | 22 | - | 91 | 0,00 | 20,1 | other |
| **NC70** | f | 31 | 113 | 5,8 | 25 | - | 107 | 3,70 | -2,8 | MCD |
| **NC72** | m | 53 | 87 | 8,7 | 25 | - | 80 | 1,94 | -3,7 | MN |
| **NC73** | f | 69 | 79 | 4,8 | 27 | - | 68 | 1,18 | -4,9 | MN |
| **NC74** | m | 68 | 25 | 0,2 | 26 | - | 45 | 2,28 | 8,9 | IGAN |
| **NC75** | m | 74 | 59 | 5,9 | 27 | - | 65 | 0,00 | 2,7 | MN |
| **NC76** | f | 53 | 43 | 9,6 | 25 | - | 67 | 0,00 | 12,1 | MCD |
| **NC77** | m | 64 | 9 | 0,9 | 25 | - | 38 | 0,07 | 13,6 | Vasculitis |
| **NC78** | m | 74 | 9 | 1,2 | 14 | - | 11 | 2,93 | 1,8 | HN |
| **NC79** | f | 27 | 116 | 2,6 | 14 | - | 112 | NA | -4,1 | other |
| **NC80** | m | 64 | 85 | 0,3 | 24 | - | 71 | 0,07 | -7,0 | Vasculitis |
| **NC81** | m | 20 | 136 | 2,2 | 12 | - | 113 | 0,08 | -22,4 | MCD |
| **NC82** | f | 54 | 81 | 11,3 | 23 | - | 81 | 0,94 | 0,2 | MCD |
| **NC83** | m | 32 | 104 | 0,2 | 25 | - | 82 | 0,36 | -10,7 | Vasculitis |
| **NC86** | f | 66 | 40 | 10,9 | 36 | - | 55 | 0,05 | 4,8 | MCD |
| **NC88** | m | 58 | 80 | 0,3 | 13 | - | 70 | 0,07 | -8,9 | Vasculitis |
| **NC89** | f | 63 | 154 | 3,0 | 22 | - | 120 | 1,50 | -17,9 | MN |
| **Progressive disease** | | | | | | | | | | |
| **NC01** | m | 51 | 12 | 7,2 | 5 | HD | 7 | 9,14 | -11,9 | DN |
| **NC06** | m | 29 | 15 | 3,2 | 6 | NTX | 6 | 1,38 | -17,9 | IGAN |
| **NC29** | m | 54 | 70 | 3,3 | 29 | - | 15 | 0,96 | -22,9 | DN |
| **NC31** | m | 58 | 26 | 5,1 | 26 | - | 7 | 1,95 | -8,8 | HN |
| **NC32** | f | 47 | 54 | 1,7 | 61 | - | 14 | 1,36 | -8,1 | HN |
| **NC33** | m | 59 | 34 | 2,9 | 26 | - | 8 | 3,16 | -11,9 | unknown |
| **NC34** | m | 41 | 16 | 3,0 | 2 | HD | 8 | 3,39 | -54,6 | IGAN |
| **NC35** | m | 42 | 48 | 0,9 | 34 | - | 8 | 0,28 | -14,2 | IGAN |

(continued)

| | | | | | | | | | | |
|---|---|---|---|---|---|---|---|---|---|---|
| **Progressive disease** | | | | | | | | | | |
| **NC37** | m | 48 | 38 | 3,6 | 25 | - | 8 | 2,68 | -14,4 | IGAN |
| **NC38** | m | 20 | 96 | 1,7 | 41 | - | 14 | 3,29 | -24,2 | IGAN |
| **NC39** | f | 63 | 37 | 8,5 | 26 | - | 3 | 6,86 | -15,9 | MN |
| **NC40** | m | 54 | 52 | 1,7 | 26 | - | 30 | 1,26 | -10,4 | MPGN |
| **NC41** | m | 35 | 46 | 0,1 | 25 | - | 32 | 0,11 | -7,0 | IN |
| **NC42** | f | 20 | 47 | 1,7 | 32 | - | 40 | 0,41 | -2,7 | pFSGS |
| **NC43** | m | 31 | 55 | 4,4 | 25 | - | 44 | 2,21 | -5,4 | pFSGS |
| **NC44** | m | 43 | 57 | 4,5 | 26 | - | 38 | 5,37 | -9,0 | pFSGS |
| **NC45** | f | 64 | 30 | 3,9 | 26 | - | 9 | 0,24 | -9,6 | sFSGS |
| **NC47** | m | 50 | 47 | 6,5 | 22 | - | 32 | 7,51 | -8,2 | IGAN |

**Versican is expressed in renal epithelial cells and in fibroblasts *in vitro.*** To analyze if versican expression is cell and/or organ specific we performed real-time PCR of the versican isoforms in cultured cells of epithelial and mesenchymal origin. We identified a massive basal expression of versican isoforms V0 and V1 in primary and immortalized human proximal tubule cells (Figure 3) and in human skin fibroblasts. Other cells such as foreskin fibroblasts, smooth muscle cells, prostate epithelial cells and colon epithelial cells showed a versican expression which was 100-1000 times less than in the kidney epithelial cells. Interestingly, whole kidney tissues from healthy controls did not show V0 and V1 expression, also pointing towards the use of VCAN for diagnosing chronic kidney disease at early stage. The levels of versican isoform V2 were extremely low in all cell lines studied, in particular in all renal epithelial cells. Although we detected some differences in the expression of the versican isoform V3, the differences were not statistically significant between the cells lines. We did not detect any of the versican isoforms in human endothelial cells. These data suggest a cell specific and probably an organ specific expression of the versican isoforms, and they represent preliminary results which are the basis for further studies of versican expression and regulation in kidney cells.

## Discussion

The novel biomarker candidates for identifying and monitoring progressive chronic kidney disease have the potential to predict the course of CKD already at an early stage when kidney function is close to normal or only slightly impaired. This information could be used to decide whether more aggressive therapies - stronger blood pressure lowering, higher doses of RAAS blockade, intensified immunosuppression - are of potential benefit for the individual patient. On the other hand the harms and benefits of such intensified therapeutic options should be carefully weighted in patients showing low biomarker expression levels thus having a potentially benign course of disease.

Using a bioinformatics analysis procedure of differential gene expression data we identified versican as a biomarker for histopathological damage in healthy kidneys, kidney grafts and in proteinuric kidney disease. In a second step we analysed the expression of versican in an independent cohort of 37 patients with various proteinuric kidney diseases and well-defined postbioptical clinical course with a median follow up time of 25 months (2-81 months; patients who were not on dialysis at end of follow-up had a follow-up time of 12-81 months). Two isoforms of versican (0 and 1) were significantly upregulated in those patients who showed a progressive loss of kidney function, suggesting that versican might serve as a potential predictive biomarker for progressive renal failure already at time of biopsy.

Versican is an extracellular matrix protein, which belongs to the family of hyaluronan-binding proteoglycans that include aggrecan, neurocan and brevican. These proteins have been grouped together on the basis of their structural similarity, and their ability to bind to the glycosaminoglycan (GAG) hyaluronan. This specific feature has also led to the collective term "hyalectins". Each of the members shows a specific tissue distribution with aggrecan being mainly expressed in cartilage, and neurocan and brevican being confined to central nervous tissue. In contrast to these rather restricted expression patterns, versican appears to show a much wider tissue distribution with expression in a variety of soft tissues. The gene and protein structure of the hyalectins show highly conserved N- and C-terminal domains: The globular amino-terminal domain (G1) is responsible for binding to hyaluronan (sometimes called "hyaluronan binding region - HABR"), while the C-terminal domain resembles the selectin family of the proteins consisting of C-type lectin, two epidermal growth factor (EGF)-like domains and a complement regulatory region (often called the "EELC domain", or G3 domain). The middle GAG binding region, however, shows little resemblance between the members of this family of proteins. While aggrecan contains up to 100 GAG side chains attached to this region, brevican, neurocan and versican contain only few chondroitin side chains. To date five isoforms of versican (V0, V1, V2, V3 and Vint) have been identified, which in most (V0, V1, V2, V3) but not all (Vint) cases result from alternative splicing of the two central exons 7 and 8 encoding the central glycosaminoglycan carrying regions, glycosaminoglycan alpha and beta (Dours-Zimmermann et al J Biol Chem 1994; 269: 32992-32998). The isoform V0 is the largest splice variant containing the N-terminal domain, both GAG-domains and the C-terminal EELC domain. The isoform V1 contains the GAG-beta but not the GAG-alpha and the isoform V2 contains the GAG-alpha but not the GAG-beta domain. The isoform V3 lacks both GAG domains resulting in no GAG attachement sites and therefore no GAG side chains. The size of the respective isoforms is predicted to be approximately 370 kDa for V0, 265 kDa for V1, 182 for V2 and 74 kDa for V3. Vint resembles an incomplete splice variant which probably retains the final intron in the carboxyterminal end of the protein. This isoform was identified by Lemire et al. (Lemire JM et al Arterioscler Thromb Vasc Biol 1999; 19: 1630-1639) and its characteristics as well as pathophysioloigical role are unclear.

Versican is expressed in healthy adult kidneys only at low levels. In chronic kidney disease increased versican expression is found in renal tissue with higher histopathological damage scores. Renal versican V0 or V1 mRNA expression is significantly higher in patients showing a progressive course of CKD than in patients with stable renal function. We demonstrated the propensity of this biomarker on the level of mRNA, indicating the propensity also on the level of protein.

**Example 3: VCAN mRNA expression in a glomerulonephritis mouse model Glomerulonephritis mouse model**

Eight- to twelve-wk-old male C57Bl/6J mice obtained from Charles River (Sulzfeld, Germany) were used throughout the studies. Animals were maintained in a virus/antibody-free central animal facility of the Innsbruck Medical University. Accelerated anti-GBM nephritis was induced as described previously (Rosenkranz, J Clin Invest 103:649-659, 1999). In brief, mice were subcutaneously pre-immunized with 2 mg/ml rabbit IgG (Jackson ImmunoResearch Laboratories, West Grove, PA) dissolved in incomplete Freund's adjuvant (Sigma, St. Louis, MI) and nonviable desiccated *Mycobacterium tuberculosis* H37a (Difco Laboratories, Detroit, MI). After 5 d, heat-inactivated rabbit anti-mouse GBM antiserum was injected via the tail vein. All animal experiments were approved by Austrian veterinary authorities. The animals were sacrificed after 14 days, the kidneys were procured and RNA was extracted as stated above.

**Versican as a marker of renal injury in the glomerulonephritis mouse model**

The resulting nephrotoxic nephritis is characterized by significant proteinuria but only slight creatinine elevation. Histological changes consisted of focal mesangial hypercellularity, focal and mild deposits of $PAS^+$ hyaline material in lumina and increases in mesangial matrix occurring in less than 10% of glomeruli, and a mild focal interstitial mononuclear cell infiltrate. We analysed the expression of mouse Versican at day 0 (controls), after 7 and after 14 days. We did not detect any significant Versican upregulation after 7 days, but there was a strong and significant upregulation of Versican after 14 days (Kruskal Wallis test p-value: 0.016) (Figure 4).

**Example 4: VCAN protein expression in human renal biopsies Immunohistochemistry and immunofluorescence**

Frozen sections of representative stable and progressive subjects were stained for human Versican protein. The sections were fixed in cold acetone and incubated at room temperature for 60 min with a 1:400 dilution of the primary antibody (rabbit antihuman Versican, Santa Cruz, sc-25831, Santa Cruz, CA, USA). Versican was detected by the Vectastain Elite ABC Kit (Vector Laboratories, Burlingame, CA, USA, www.vectorlabs.com) and stained with 3-amino-9-ethylcarbazole. This system uses a biotin-conjugated secondary antibody (1:1000), avidin and biotinylated horseradish peroxidase and the corresponding chromogen for visualization. All sections were counterstained with 3,30-diaminobenzidine tetrahydrochloride 3-amino-9-ethyl carbazole.

Versican protein was expressed in several compartments of the kidney biopsies. The weakest expression was found in the glomeruli (Figure 5 A), while the strongest expression was found in the tubulinterstitial compartment, both in tubuli (Figure 5 B) and in the interstitial fibroblasts and in areas of fibrosis (Figure 5 C). Interestingly, Versican was also expressed in the media of some but not all renal cortical blood vessels (Figure 5 D).

Versican, thus, qualifies as a marker of renal disorders. The differentiation between the versican isoforms and the specific determination of the inventive V0 and/or V1 will improve the determination of the risk of renal disorders, including the determination of renal disease.

## Claims

1. An in vitro method of determining the risk of renal disorders in a patient, wherein upregulation of at least one of the VCAN isoforms V0 and V1 is determined in a sample of said patient and compared to a reference level.

2. A method according to claim 1, wherein said renal disorders are selected from acute, chronic, proteinuric or progressive kidney disease and associated increased morbidity or mortality from cardiovascular disease and bone metabolism disorders.

3. The method according to claims 1 or 2, wherein the increase is at least 1.5 times the reference value of subjects not at risk of the renal disorder.

4. The method according to any one of claims 1 to 3, wherein said sample is a tissue, blood, serum, plasma , urine or a kidney biopsy sample.

5. The method according to any one of claims 1 to 4, wherein the expression of VCAN isoforms V0 and V1 is determined.

6. The method according to any one of claims 1 to 5, wherein upregulation is determined by microarray hybridization with specific probes or by PCR.

7. The method according to any one of claims 1 to 6, wherein a further kidney risk factor (KRF) or senescence parameter is measured.

8. The method according to any one of claims 1 to 7, comprising

    (a) contacting a sample obtained from said patient with oligonucleotides that specifically hybridize to the V0 and/or V1 isoform(s), and
    (b) detecting in the sample a level of polynucleotides that hybridize to the isoform(s) relative to a predetermined cut-off value, and therefrom determining the risk of renal disorders in the patient.

9. The method according to any one of claims 1 to 7, comprising comparing:

    (a) levels of the V0 and/or V1 isoform(s) in a sample from said patient, and
    (b) normal levels of said isoform(s) in samples of the same type obtained from control patients, wherein altered levels of the isoform(s) relative to the corresponding normal levels is an indication that the patient has a risk of renal disorder.

10. The method according to any one of claims 1 to 7, comprising quantitating the V0 and/or V1 isoform(s) in a sample from said patient by a method comprising

    (a) reacting the sample with one or more binding agents specific for the isoform(s) labelled with a detectable substance, and
    (b) detecting the detectable substance.

11. The method according to any one of claims 1 to 7, comprising assessing the potential of a test compound to contribute to kidney disease or onset of kidney disease by a method comprising:

    (a) maintaining separate aliquots of a sample from a patient in the presence and absence of the test compound, and
    (b) comparing the levels of the V0 and/or V1 isoform(s) in each of the aliquots.

12. Use of a method according to any one of claims 1 to 11, for the monitoring of a patient, for the prognosis of kidney failure or for the diagnosis of renal disease in a patient at risk of disease progression.

## Patentansprüche

1. In vitro-Verfahren zum Bestimmen der Gefahr von Nierenstörungen bei einem Patienten, wobei die Hochregulierung von mindestens einem der VCAN-Isoformen V0 und V1 in einer Probe des Patienten bestimmt und mit einem Referenzpegel verglichen wird.

2. Verfahren nach Anspruch 1, wobei die Nierenstörungen ausgewählt werden aus akuter, chronischer, Proteinurie- oder progressiver Nierenkrankheit und zugehöriger erhöhter Morbidität oder Mortalität durch kardiovaskuläre Krankheit und Knochenmetabolismus-Störungen.

3. Verfahren nach Anspruch 1 oder 2, wobei die Erhöhung mindestens das 1,5fache des Referenzwertes der Menschen ist, bei denen keine Gefahr von Nierenstörungen besteht.

4. Verfahren nach einem der Ansprüche 1 bis 3, wobei die Probe eine Gewebs-, Blut-, Serum-, Plasma-, Urin-oder eine Nierenbiopsieprobe ist.

5. Verfahren nach einem der Ansprüche 1 bis 4, wobei die Expression der VCAN-Isoformen V0 und V1 bestimmt wird.

6. Verfahren nach einem der Ansprüche 1 bis 5, wobei die Hochregulierung durch Mikroarray-Hybridisierung mit speziellen Sonden oder durch PCR bestimmt wird.

7. Verfahren nach einem der Ansprüche 1 bis 6, wobei ein weiterer Nierenrisikofaktor (KRF) oder Seneszenz-Parameter gemessen wird.

8. Verfahren nach einem der Ansprüche 1 bis 7, umfassend

(a) Kontaktieren einer Probe, die von dem Patienten gewonnen wurde, mit Oligonukleotiden, die spezifisch mit den V0- und/oder V1-Isoformen hybridisieren, und
(b) in der Probe, Feststellen eines Pegels von Polynukleotiden, die mit der/den Isoform(en) relativ zu einem vorgegebenen Schwellenwert hybridisieren, und daraus die Gefahr von Nierenstörungen bei dem Patienten bestimmen.

9. Verfahren nach einem der Ansprüche 1 bis 7, das das Vergleichen umfasst:

(a) Spiegel der V0- und/oder V1-Isoform(en) in einer Probe von dem Patienten, und
(b) normale Spiegel der Isoform(en) in Proben desselben Typs, die von Kontrollpatienten erhalten werden, wobei geänderte Spiegel der Isoform(en) relativ zu den entsprechenden normalen Spiegeln ein Anzeichen dafür sind, dass bei dem Patienten eine Gefahr von Nierenstörungen besteht.

10. Verfahren nach einem der Ansprüche 1 bis 7, das das Quantifizieren der V0- und/oder V1-Isoform(en) in einer Probe von dem Patienten durch ein Verfahren umfasst, welches enthält

(a) Umsetzen der Probe mit einem oder mehreren Bindemitteln, die spezifisch für die Isoform(en) sind, welche(r) mit einer nachweisbaren Substanz markiert sind, und
(b) Nachweisen der nachweisbaren Substanz.

11. Verfahren nach einem der Ansprüche 1 bis 7, das das Bewerten des Potenzials einer Testverbindung, die zu einer Nierenkrankheit oder dem Einsetzen einer Nierenkrankheit beiträgt, durch ein Verfahren umfasst, das enthält:

(a) Aufrechterhalten von getrennten Teilproben einer Probe von einem Patienten bei Anwesenheit und bei Fehlen der Testverbindung, und
(b) Vergleichen der Spiegel der V0- und/oder V1-Isoform(en) in jeder der Teilproben.

12. Verwendung eines Verfahrens nach einem der Ansprüche 1 bis 11 zum Überwachen eines Patienten für die Prognose von Nierenversagen oder für die Diagnose von Nierenkrankheit in einem Patienten, bei dem eine Gefahr von Krankheitsfortschritt besteht.

**Revendications**

1. Procédé in vitro de détermination du risque de troubles rénaux chez un patient, dans lequel la régulation positive d'au moins une des isoformes de VCAN V0 et V1 est déterminée dans un échantillon dudit patient et comparée à un niveau de référence.

2. Procédé selon la revendication 1, dans lequel lesdits troubles rénaux sont choisis parmi une maladie du rein aiguë, chronique, protéinurique ou progressive et la morbidité ou la mortalité accrue associée d'une maladie cardiovasculaire et des troubles du métabolisme de l'os.

3. Procédé selon la revendication 1 ou 2, dans lequel l'augmentation est d'au moins 1,5 fois la valeur de référence de sujets non à risque du trouble rénal.

4. Procédé selon l'une quelconque des revendications 1 à 3, dans lequel ledit échantillon est un tissu, du sang, du sérum, du plasma, de l'urine ou un échantillon de biopsie de rein.

5. Procédé selon l'une quelconque des revendications 1 à 4, dans lequel l'expression des isoformes de VCAN V0 et V1 est déterminée.

6. Procédé selon l'une quelconque des revendications 1 à 5, dans lequel la régulation positive est déterminée par hybridation sur microréseau avec des sondes spécifiques ou par PCR.

7. Procédé selon l'une quelconque des revendications 1 à 6, dans lequel un autre facteur de risque rénal (KRF) ou paramètre de sénescence est mesuré.

8. Procédé selon l'une quelconque des revendications 1 à 7, comprenant

(a) la mise en contact d'un échantillon obtenu dudit patient avec des oligonucléotides qui s'hybrident spécifiquement à l'(aux) isoforme(s) V0 et/ou V1, et
(b) la détection dans l'échantillon d'un niveau de polynucléotides qui s'hybrident à (aux) l'isoforme(s) relativement à une valeur seuil prédéterminée, et ainsi déterminer le risque de troubles rénaux chez un patient.

9. Procédé selon l'une quelconque des revendications 1 à 7, comprenant la comparaison :

(a) des niveaux de l'(des) isoforme(s) V0 et/ou V1 dans un échantillon dudit patient, et
(b) des niveaux normaux de ladite(desdites) isoforme(s) dans des échantillons du même type obtenus de patients témoins, où les niveaux modifiés de l'(des) isoforme(s) relativement aux niveaux normaux correspondants est une indication que le patient a un risque de trouble rénal.

10. Procédé selon l'une quelconque des revendications 1 à 7, comprenant la quantification de l'(des) isoforme(s) V0 et/ou V1 dans un échantillon dudit patient par un procédé comprenant

(a) la réaction de l'échantillon avec un ou plusieurs agents de liaison spécifiques de l'(des) isoforme(s) marquée(s) avec une substance détectable, et
(b) la détection de la substance détectable.

11. Procédé selon l'une quelconque des revendications 1 à 7, comprenant l'évaluation du potentiel d'un composé test pour contribuer à une maladie rénale ou au déclenchement d'une maladie rénale, par un procédé comprenant :

(a) le maintien d'aliquotes séparées d'un échantillon d'un patient en présence ou en absence du composé test, et
(b) la comparaison des niveaux de l'(des) isoforme(s) V0 et/ou V1 dans chacune des aliquotes.

12. Utilisation d'un procédé selon l'une quelconque des revendications 1 à 11, pour le suivi d'un patient, pour le pronostic d'une insuffisance rénale ou pour le diagnostic d'une maladie rénale chez un patient à risque de la progression de la maladie.

Fig 1

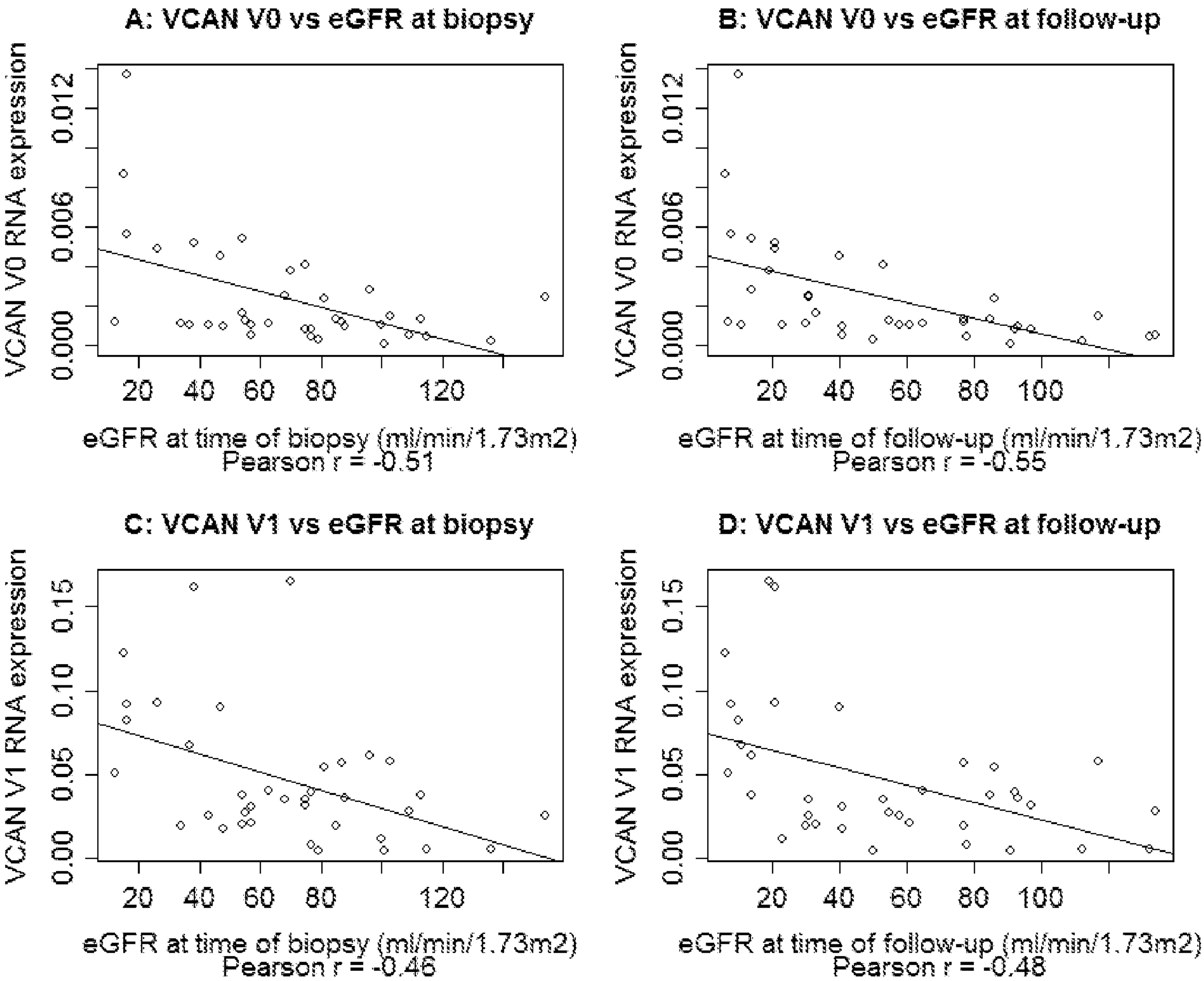
A: VCAN V0 vs eGFR at biopsy
VCAN V0 RNA expression
0.000
0.006
0.012
20 40 60 80 120
eGFR at time of biopsy (ml/min/1.73m2)
Pearson r = -0.51
B: VCAN V0 vs eGFR at follow-up
VCAN V0 RNA expression
0.000
0.006
0.012
20 40 60 80 100
eGFR at time of follow-up (ml/min/1.73m2)
Pearson r = -0.55
C: VCAN V1 vs eGFR at biopsy
VCAN V1 RNA expression
0.00
0.05
0.10
0.15
20 40 60 80 120
eGFR at time of biopsy (ml/min/1.73m2)
Pearson r = -0.46
D: VCAN V1 vs eGFR at follow-up
VCAN V1 RNA expression
0.00
0.05
0.10
0.15
20 40 60 80 100
eGFR at time of follow-up (ml/min/1.73m2)
Pearson r = -0.48

Fig. 2

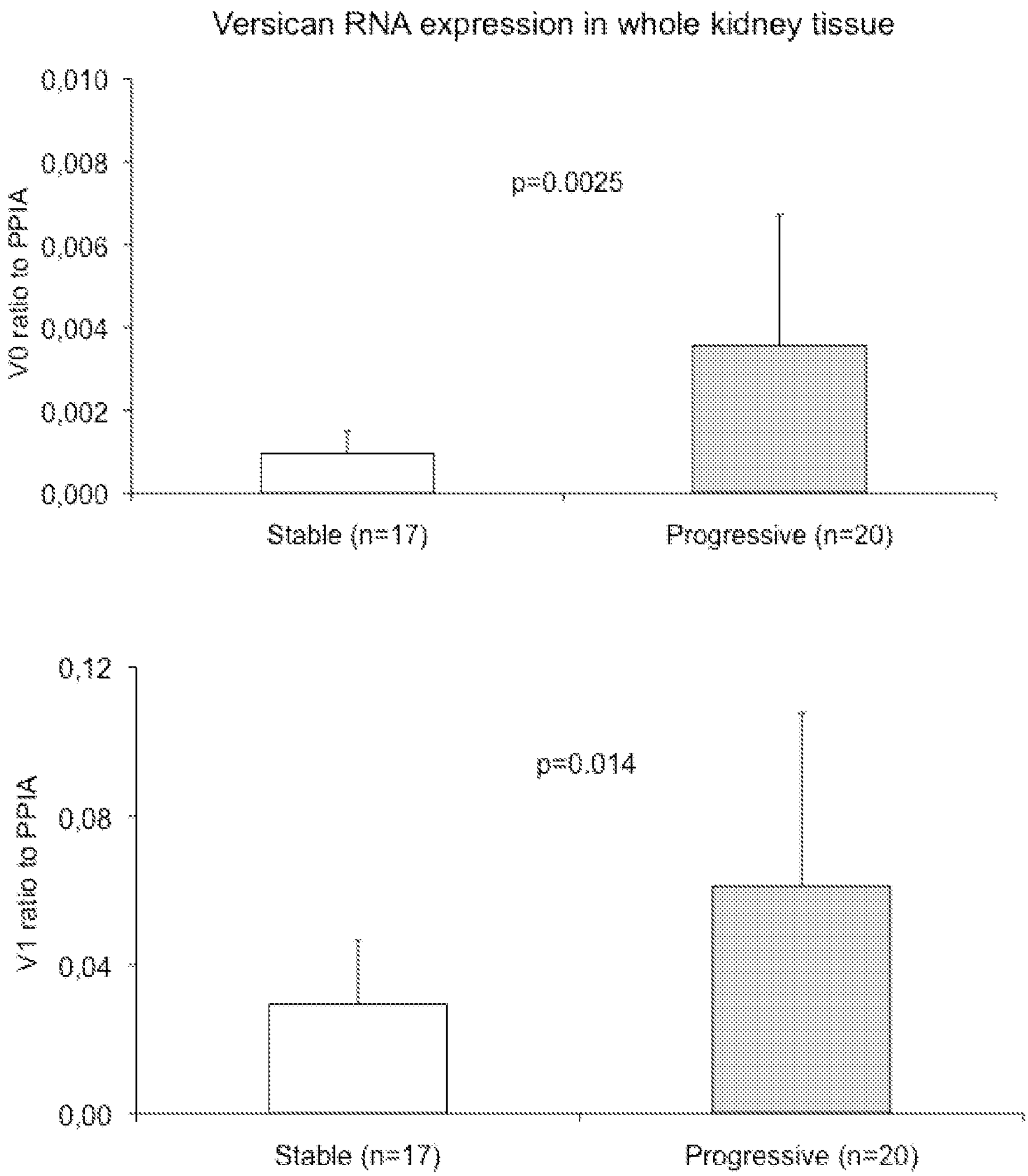
Versican RNA expression in whole kidney tissue
V0 ratio to PP1A
0,010
0,008
0,006
0,004
0,002
0,000
p=0.0025
Stable (n=17)
Progressive (n=20)
V1 ratio to PPIA
0,12
0,08
0,04
0,00
p=0.014
Stable (n=17)
Progressive (n=20)

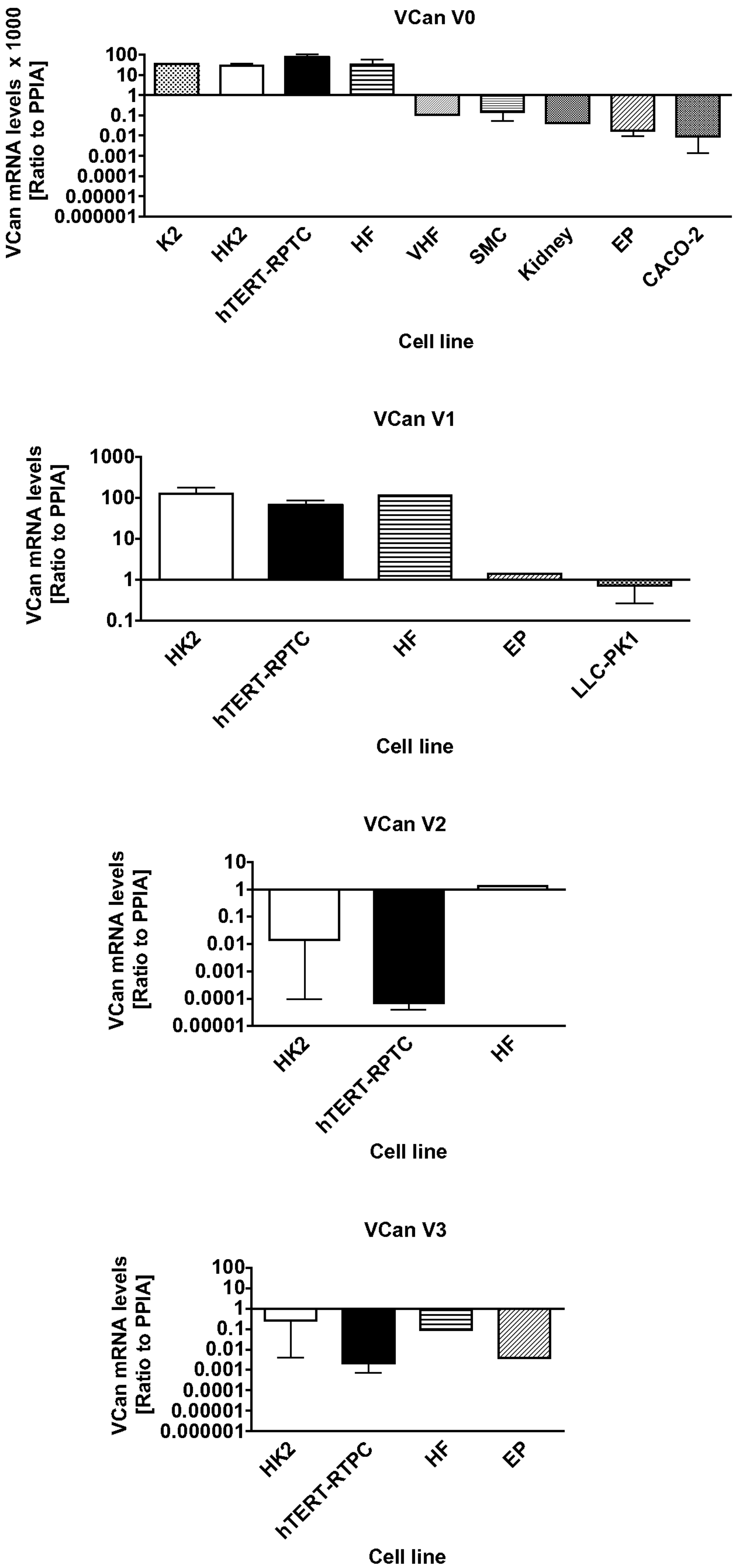
VCan V0
VCan mRNA levels x 1000
[Ratio to PPIA]
100
10
1
0.1
0.01
0.001
0.0001
0.00001
0.000001
K2
HK2
hTERT-RPTC
HF
VHF
SMC
Kidney
EP
CACO-2
Cell line
VCan V1
VCan mRNA levels
[Ratio to PPIA]
1000
100
10
1
0.1
HK2
hTERT-RPTC
HF
EP
LLC-PK1
Cell line
VCan V2
VCan mRNA levels
[Ratio to PPIA]
10
1
0.1
0.01
0.001
0.0001
0.00001
HK2
hTERT-RPTC
HF
Cell line
VCan V3
VCan mRNA levels
[Ratio to PPIA]
100
10
1
0.1
0.01
0.001
0.0001
0.00001
0.000001
HK2
hTERT-RTPC
HF
EP
Cell line

Fig. 3

Fig. 4

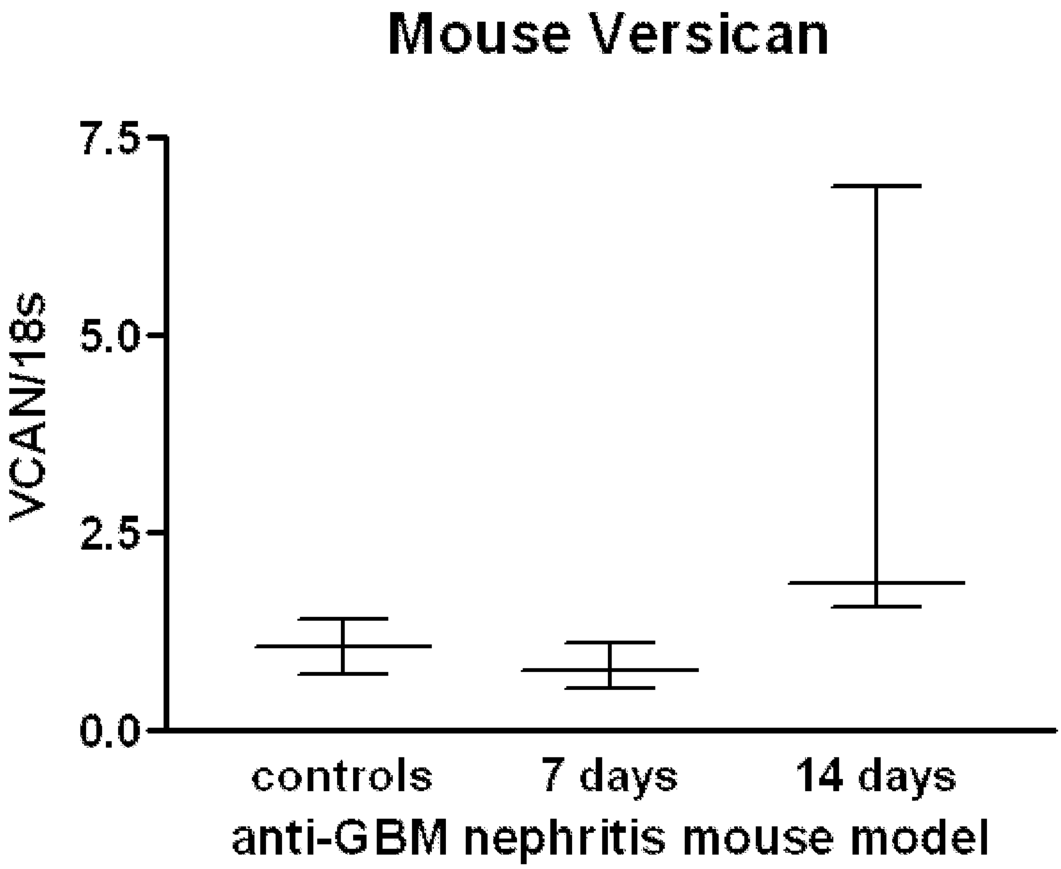


Kruskal-Wallis p=0.016

Fig. 5

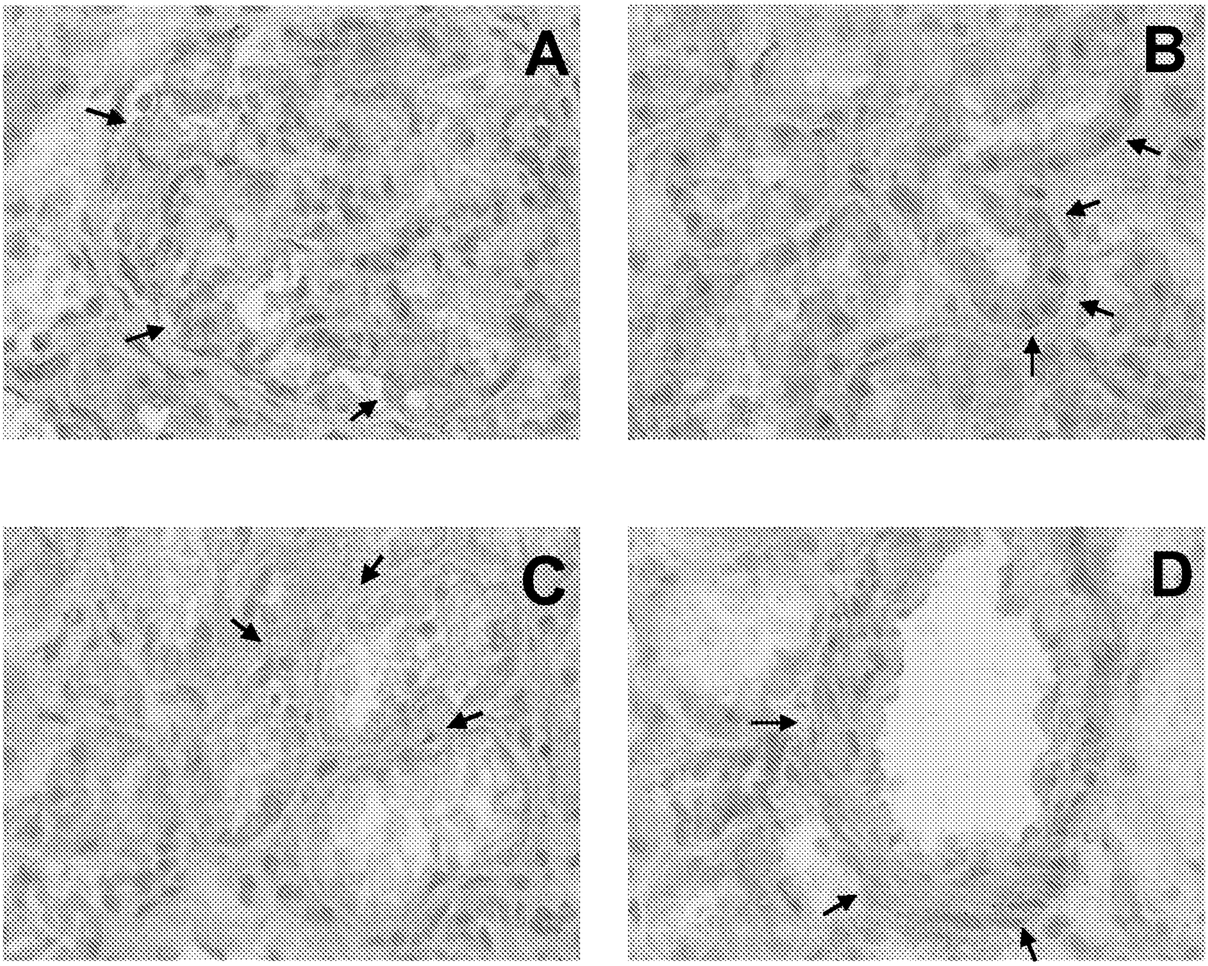

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2008068083 W
- EP 2009054439 W
- WO 2007096142 A2
- WO 2009061368 A
- WO 9108230 A


### Non-patent literature cited in the description


- **RUDNICKI et al.** *Nephron Exp Nephrol,* 2004, vol. 97, e86-e95
- **RUDNICKI et al.** *Kidney International,* 2007, vol. 71, 325-335
- **PERCO et al.** *European Journal of Clinical Investigation,* 2006, vol. 36, 753-763
- **STOKES et al.** *Kidney International,* 2001, vol. 59 (2), 532-542
- **DOURS-ZIMMERMANN et al.** *The Journal of Biological Chemistry,* 1994, vol. 269 (52), 32992-32998
- **CATTARUZZA et al.** *Journal of Biological Chemistry,* 2002, vol. 277 (49), 47626-47635
- **ARSLAN et al.** *British Journal of Cancer,* 2007, vol. 96 (10), 1560-1568
- **DOURS-ZIMMERMANN et al.** *J Biol Chem,* 1994, vol. 269, 32992-32998
- **LEMIRE JM et al.** *Arterioscler Thromb Vasc Biol,* 1999, vol. 19, 1630-1639
- **ROSENKRANZ.** *J Clin Invest,* 1999, vol. 103, 649-659